# EUROPÄISCHE PATENTANMELDUNG

(11) **EP 4 609 780 A1**
(43) Veröffentlichungstag der Anmeldung: **03.09.2025**
(21) Anmeldenummer: 24160933.8
(22) Anmeldetag: 01.03.2024
(51) Int. Cl.: A61B 3/028, A61B 3/103, A61B 3/00

(54) **VERFAHREN ZUM ÜBERPRÜFEN ZUMINDEST EINES SEHEINDRUCKS EINES PROBANDEN, SEHPRÜFSYSTEME, EIN LINSENTRÄGER, EIN COMPUTERPROGRAMMPRODUKT SOWIE EIN COMPUTERLESBARES SPEICHERMEDIUM**

(71) Anmelder: Dezimal GmbH, 1100 Wien (AT)
(72) Erfinder: Kornfeld, Martin, 1100 Wien (AT); La Schiazza, Olivier, 69120 Heidelberg (DE); Dworschak, Rüdiger, 67146 Deidesheim (DE)
(74) Vertreter: Neuhold, Alfred

(57) **Zusammenfassung**

Die Erfindung betrifft ein Verfahren zum Überprüfen zumindest eines Seheindrucks eines Probanden mit einem Sehprüfsystem, wobei das Sehprüfsystem eine Linsenträgeraufnahme zum Aufnehmen eines Linsenträgers umfasst, die in einer zentralen Sichtachse 21 des optischen Strahlengangs von distal zu proximal aufeinanderfolgend zwischen einer Cornea-Optik 40 und einer Okular-Optik 45 gelagert ist und umfasst zumindest die folgenden Schritte: (a) Positionieren eines ersten Linsenträgers mit einer Intraokularlinse in der Linsenträgeraufnahme des Sehprüfsystems; (b) Verschieben der Cornea Optik 40 und der Linsenträgeraufnahme mit der Intraokularlinse entlang der Sichtachse 21, wobei die Okular-Optik 45 in einem fixen Abstand zu zumindest einem Auge des Probanden verbleibt; (c.) Erfassen zumindest einer für den Seheindruck indikativen Information; (d.) Zuordnen der mindestens einen indikativen Information zur ersten Intraokularlinse. Weiters umfasst die Erfindung Sehprüfsysteme und einen Linsenträger.

## Beschreibung

Die vorliegende Erfindung betrifft ein Verfahren zum Überprüfen zumindest eines Seheindrucks eines Probanden gemäss dem Patentanspruch 1, Sehprüfsysteme gemäss dem Patentanspruch 12, ein Linsenträger gemäss dem Patentanspruch 13, ein Computerprogrammprodukt gemäss dem Patenanspruch 14 sowie ein computerlesbares Speichermedium gemäss dem Patentanspruch 15.

### Technologischer Hintergrund

Die Implantation einer phaken, aphaken oder pseudophaken Intraokularlinse im Auge eines Probanden ist ein überaus kritischer Prozess. Die Intraokularlinse muss bestmöglich an die individuellen Erfordernisse des Probanden angepasst sein, da jeder spätere Austausch ein unnötiges Operationsrisiko in sich birgt. Daher ist es notwendig und wünschenswert, die Sicht des Probanden durch eine zu implantierende Intraokularlinse bzw. deren optische Eigenschaften bereits vor einer Implantation gründlich zu überprüfen, um einen Austausch in vivo unter allen Umständen zu vermeiden.

Herkömmlicherweise werden dazu entweder Computersimulationen oder optische Prüfvorrichtungen, in welche die zu implantierende Intraokularlinse eingesetzt wird, verwendet. Mittels Computersimulation anhand eines Auges mit durchschnittlicher Anatomie erstellte Bilder, die dem Probanden gezeigt werden, geben jedoch stets nur sehr allgemeine Eigenschaften einer Intraokularlinse wieder. Mit Simulationen ist es daher unmöglich, den subjektiven Seheindruck des Probanden durch die für die Implantation ausgewählte Intraokularlinse zuverlässig vorherzusagen. Darüber hinaus ist es besonders schwierig, unterschiedliche Sehsituationen zuverlässig zu simulieren, z.B. verschiedene Lichtverhältnisse und Sichtweiten wie die Fernsicht beim Betrachten einer Landschaft und die Nahsicht beim Lesen eines Buches.

Optische Prüfvorrichtungen eignen sich besser zur Überprüfung des subjektiven Seheindrucks in unterschiedlichen Situationen.

Im Stand der Technik sind zwei Typen multifokaler Intraokularlinsen bekannt: ein refraktiver Typ und ein diffraktiver Typ. Der refraktive Typ einer multifokalen Intraokularlinse weist eine Linsenoberfläche auf, die aus verschiedenen Oberflächenbereichen mit unterschiedlichen Krümmungsradien besteht, der diffraktive Typ einer multifokalen Intraokularlinse weist eine diffraktive Struktur auf und jeder dieser beiden Typen multifokaler Intraokularlinsen bildet eine Vielzahl von Lichtbündelungspunkten (einen für die Fernsicht und einen für die Nahsicht und ggf. einen für die eine intermediäre Sicht) an verschiedenen Positionen in Richtung der optischen Sichtachse. Diese Linsenstruktur ermöglicht es dem Träger, entweder an einem Lichtbündelungspunkt für die Fernsicht oder an einem Lichtbündelungspunkt für die Nahsicht oder an einem Lichtbündelungspunkt für die intermediäre Sicht eine ausreichende Sehkraft zu gewährleisten, sodass der Träger alltägliche Aktivitäten ausführen kann, ohne auf eine Brille angewiesen zu sein. Die multifokale Intraokularlinse weist unterschiedliche Brechkräfte auf: eine primäre Brechkraft und mindestens eine zusätzliche Brechkraft, die der primären Brechkraft entspricht, zu der eine differenzielle Brechkraft hinzugefügt wird.

Aus dem Stand der Technik ist die US 8,042,945 B2 bekannt. Es wird ein Simulator für multifokale Intraokularlinsen bereitgestellt, mit dem man die Auswirkungen einer multifokalen Intraokularlinsenimplantation, den Unterschied zwischen multifokalen Intraokularlinsen vom diffraktiven Typ und vom refraktiven Typ sowie die Nachteile einer multifokalen Intraokularlinse tatsächlich wahrnehmen und erleben kann, ohne dass dies erforderlich ist sich einer multifokalen Intraokularlinsen-Implantation zu unterziehen, um solche Effekte wahrzunehmen/erleben zu können (d. h. vor einer multifokalen Intraokularlinsen-Implantation). Die vorliegende Erfindung stellt außerdem ein Verfahren zur Simulation einer multifokalen Intraokularlinse bereit, welches die oben beschriebenen Effekte erzielt. Um eine Fehlrefraktionskompensation oder Dioptrieneinstellung zu ermöglichen, erlaubt der Simulator eine Verschiebung der Okularlinse. Die im Simulator einsetzbare Linse erlaubt eine Nachbildung einer Intraokularlinse.

Nachteilig ist, dass die Einstellung auf verschiedene Refraktionsstufen nur mittels Anpassung einer Okularlinse stattfindet. Eine korrekte optische Abbildung bei myopen und hyperopen Augen im Vergleich zum pseudophaken Auge (Fehlrefraktion durch die implantierte IOL kompensiert) kann auf diese Art nicht sichergestellt werden.

### Darstellung der Erfindung

Eine Aufgabe der Erfindung besteht darin mindestens einen der Nachteile des Standes der Technik zu vermeiden. Es soll ein verbessertes Verfahren zum Überprüfen zumindest eines Seheindrucks eines Probanden durch eine echte Intraokularlinse mit einem Sehprüfsystem geschaffen werden, welches eine qualitativ verbesserte Auswahl, einer, für den spezifischen Probanden geeigneten, echten Intraokularlinse ermöglicht. Weiters sollen verbesserte Sehprüfsysteme geschaffen werden, welche die verbesserte Auswahl einer für den spezifischen Probanden geeigneten, echten Intraokularlinse ermöglicht bzw. mit welcher die Auswahl verifizierbar ist. Darüber hinaus soll ein Linsenträger mit einer Intraokularlinse geschaffen werden, der unverwechselbar mit der durch den spezifischen Probanden ausgewählten Intraokularlinse verbindbar ist.

Diese Aufgabe wird durch die Merkmale der unabhängigen Patentansprüche gelöst. Vorteilhafte Weiterbildungen sind in den Figuren und in den abhängigen Patentansprüchen dargelegt.

Ein erfindungsgemässes Verfahren zum Überprüfen zumindest eines Seheindrucks eines Probanden mit einem Sehprüfsystem, wobei das Sehprüfsystem eine Linsenträgeraufnahme zum Aufnehmen zumindest eines Linsenträgers umfasst, die in einer zentralen Sichtachse des optischen Strahlengangs von distal (nahe zur betrachteten Umgebung) zu proximal (nahe zum Auge des Probanden) aufeinanderfolgend zwischen einer Cornea-Optik und einer Okular-Optik gelagert ist, umfasst zumindest die folgenden Schritte:
a. Positionieren eines ersten Linsenträgers mit einer ersten Intraokularlinse in der Linsenträgeraufnahme des Sehprüfsystems;
b. Verschieben der Cornea-Optik und der Linsenträgeraufnahme mit der Intraokularlinse entlang der zentralen Sichtachse, wobei die Okular-Optik in einem fixen Abstand zu zumindest einem Auge des Probanden verbleibt;
c. Erfassen zumindest einer für den Seheindruck indikativen Information;
d. Zuordnen der mindestens einen indikativen Information zur ersten Intraokularlinse.

Das Verfahren ermöglicht es beispielsweise einem presbyopen Probanden reale Gegenstände in der für das funktionelle Sehen relevanten Nah-, Intermediär- und Ferndistanz in einem ersten qualitativen Sehtest zu erfassen, um seine Wahrnehmung mit einer bestimmten, echten Intraokularlinse im Gesamtbild qualitativ in Abhängigkeit der indikativen Information zu bewerten und so die individuell beste Intraokularlinse im Sinne seiner subjektiven Beurteilung zu finden. Schlussendlich kann der Proband selbst die für ihn subjektiv geeignetste Intraokularlinse beurteilen. Dabei kann der Proband zwischen z.B. monofokaler, bifokaler, multifokaler oder EDOF-Linsen als Intraokularlinsen wählen. Der Proband erlebt den gleichen Seheindruck, als wäre die Intraokularlinse bereits in seinem Auge implantiert. Die indikative Information umfasst nicht abschliessend aufgezählt zumindest einen der nachfolgenden Parameter, nämlich Kontrast, Schärfe, Farbe, Streulichteffekte, Lichtsensation, oder Dysphotopsie. Auf eine Simulation von Parametern zur Intraokularlinse kann verzichtet werden.

Ein wesentlicher Vorteil von diesem System ist, dass die korrekte optische Abbildung bei myopen und hyperopen Augen von Probanden im Vergleich zum pseudophaken Auge erhalten bleibt.

Alternativ oder ergänzend erfolgt im Schritt b. ein Einstellen der Brechkraft mit einer einstellbaren Linse. Damit kann auf ein Verschieben der Cornea-Optik und der Linsenträgeraufnahme weitgehend verzichtet werden. Danach können die Schritte c. und d. erfolgen. Eine einstellbare Linse kann beispielsweise eine fokussierbare Flüssiglinse oder eine Alvarez-Humphrey-Linse sein.

Insbesondere können die hier vorliegend beschriebenen Optiken bzw. Linsen aus einer Linse bestehen oder auch mehrere Linsen umfassen, um die gewünschten optischen Effekte zu ermöglichen. Beispielsweise kann eine hier beschriebene Kompensationslinse oder, eine hier beschriebene Intraokularlinse oder eine hier beschriebene Korrekturlinse oder eine hier beschriebene einstellbare Linse aus einer oder mehreren Linsen bestehen.

Insbesondere umfasst die Okular-Optik distal eine Korrekturlinse und, proximal dazu, eine Zerstreuungslinse und eine Sammellinse, wobei die Korrekturlinse eine distale konkave Linsenoberfläche und eine proximale konvexe Linsenoberfläche aufweist. Dies erlaubt durch die spezielle Form der Linsenoberflächen, ein Verfahren mit einem Sehprüfsystem, bei dem das Sichtfeld der Vorrichtung bzw. das Sichtfeld des durch diese blickenden Auges eines Probanden erweitert ist und gleichzeitig Defokussierungen und Kontrastfehler im erweiterten Sichtfeld korrigierbar sind und bestenfalls eliminierbar sind.

Der hier vorliegend beschriebene Proband kann auch als Patient bezeichnet werden, insbesondere wird er als Patient bei post-operativen Überprüfungsverfahren genannt.

Das zuvor genannte Verfahren kann wiederholt werden, wobei im Schritt a. ein zweiter Linsenträger mit einer zweiten Intraokularlinse anstelle des ersten Linsenträgers in der Linsenträgeraufnahme des Sehprüfsystems positioniert wird. Damit kann der Proband bei einem qualitativen Sehtest die für ihn geeignete echte Intraokularlinse subjektiv auswählen. Sollte auch die zweite Intraokularlinse vom Probanden als ungeeignet empfunden werden, so kann der Linsenträger mit der zweiten Intraokularlinse durch einen weiteren Linsenträger mit einerweiteren Intraokularlinse getauscht werden und das zuvor genannte Verfahren wiederholt werden.

Vor dem Schritt a. kann der Proband korrekt auf das Sehprüfsystem eingestellt werden, so dass zumindest ein Auge des Probanden auf der zentralen Sichtachse des jeweiligen optischen Strahlengangs zentriert ist. Dies erfolgt einerseits durch eine höhenverstellbare Kinn- oder Kopfstütze zur vertikalen Positionierung des Kopfes des Probanden und andererseits durch Verstellen des Abstands zumindest eines optischen Strahlengangs im Sehprüfsystem auf die Pupillendistanz der Augen des Probanden. Alternativ oder ergänzend dazu kann auch die Position der Optik vertikal auf die Position des Auges des Probanden eingestellt werden. In einem weiteren Schritt kann ein eventuell vorhandener regulärer Astigmatismus im Auge des Probanden durch zylindrische Vorsatzlinsen oder einer Stokes-Linse oder einer Alvarez-Humphrey-Linse kompensiert werden. Die zylindrische Vorsatzlinsen (oder Stokes-Linse oder Alvarez-Humphrey-Linse) können in der entsprechenden Stärke und Achslage in den optischen Strahlengang des Sehprüfsystems für die jeweilige Augenseite positioniert werden. Die Stokes-Linse oder Alvarez-Humphrey-Linse kann auch in das Gerät integriert sein.

Zum Einstellen einer eventuell vorhandenen sphärischen Fehlrefraktion des Auges des Probanden, kann durch axiales Verschieben einer Optik (Linse oder Linsengruppe), insbesondere der Cornea-Optik, im optischen Strahlengang die Brechkraft des gesamten optischen Systems verändert werden. Bestehende Refraktionsfehler des Auges werden auf diese Weise kompensiert und ermittelt.

Die Kompensation der eventuell vorhandenen Refraktionsfehler des Auges des Probanden durch internes Verschieben zumindest einer Linsen (-gruppe) im optischen Strahlengang, erlaubt es für unterschiedliche Probanden den gleichen Linsenträger mit der gleichen ersten Intraokularlinse in vordefinierter, gleichbleibender Brechkraft (z.B. 20,0 Dioptrien) zu verwenden. Weiter erlaubt das interne Verändern der Brechkraft des Gesamtsystems, bei fest vorgegebener Nah- und Intermediärdistanz von Objekt zum Sehprüfsystem, das Bild durch Intraokularlinsen mit unterschiedlichen Additionen auf der Netzhaut des Probanden scharf abzubilden.

Alternativ oder ergänzend kann die Brechkraft des gesamten optischen Systems mit einer einstellbaren Linse (z.B. fokussierbare Flüssiglinse oder Alvarez-Humphrey-Linse) im optischen Strahlengang, eingestellt werden.

Ein im optischen Strahlengang der jeweiligen Augenseite eingesetzter Shutter, ermöglicht, zwischen monokularem und binokularem Blick durch das Sehprüfsystem zu wechseln.

Vorzugsweise wird mindestens ein Datenwert, der während eines ersten Sehtests mit einem Objekt repräsentativ ist, gemessen. Der Proband blickt dabei durch die zuvor ausgewählte Intraokularlinse auf zumindest einen Optotypen. Ein Optotyp ist hier vorliegend ein Sehzeichen, ein Objekt, eine Graphik, oder auch eine Abbildung. Beispielsweise umfasst der erste Sehtest einen Visus-Test, bei dem Optotypen in verschieden Grössen und Orientierungen resp. Abfolgen an einer Anzeigeeinrichtung des Sehprüfsystems angezeigt werden. Dabei werden Sehschärfewerte vom durch die Intraokularlinse blickenden Probanden gemessen. Ein Sehschärfewert gilt beispielsweise als erreicht, wenn mindestens 3 von 5 Optotypen einer Visus-Stufe als korrekt erkannt werden.

Insbesondere wird der mindestens eine Datenwert der ersten Intraokularlinse zugeordnet. Dabei können beispielsweise verschieden Grössen und Orientierungen resp. Abfolgen der Optotypen, oder ein ermittelter Sehschärfewert, der ersten Intraokularlinse zugeordnet werden. Werden mehrere Datenwerte erfasst, entsteht ein Datensatz von Datenwerten, welche die vorliegend getestete erste Intraokularlinse mit dem Auge des Probanden verlinkt. Der Datensatz kann bei der schlussendlichen Auswahl der Intraokularlinse für eine Auswertung bzw. für einen Vergleich von Datensätzen unterschiedlicher Intraokularlinsen herangezogen werden. Der Datensatz kann wiederkehrend verwendet werden, und wird beispielsweise bei post-operativen Sehtests zur Dokumentation und Qualitätssicherung der Güte der Intraokularlinsen-Implantation herangezogen. Postoperativ benötigt man vorteilhaft die Datenwerte der Intraokularlinse, insbesondere die Datenwerte der Addition. Der Refraktionsfehler ist i.d.R. durch die implantierte Intraokularlinse kompensiert.

Bevorzugterweise umfasst das Sehprüfsystem eine Steuerungseinrichtung sowie insbesondere der erste Linsenträger ein Speichermedium, wobei ein erster Parameter von dem Speichermedium des ersten Linsenträgers an die Steuerungseinrichtung übermittelt wird. Die Steuerungseinrichtung kann den ersten Parameter als Grundlage für einen Steuerbefehl heranziehen, um beispielsweise einen invaliden Linsenträger zu erkennen, und beispielsweise ein Warnsignal an einer Anzeigeeinrichtung des Sehprüfsystems ausgeben zu können. Dabei umfasst der erste Parameter zumindest eine der folgenden Informationen, nämlich Parameter zur Echtheit bzw. Validität des Linsenträgers (Zertifikat), Name, Artikelnummer, Herstelldatum, Haltbarkeit, Seriennummer und Position im Strahlengang, Justagewerte, Kalibrierwerte, einen Fokuspunkt in Abhängigkeit von der Addition in der ersten Intraokularlinse. Damit ist die Systemintegrität sowie die Prozessintegrität verifizierbar und eine Fehlbedienung im Verfahren vermeidbar. Somit kann eine falsche Zuordnung einer Intraokularlinse vermieden werden. Insbesondere umfasst der erste Linsenträger mehrere der zuvor gelisteten Parameter, um eine höhere Spezifizierung der ersten Intraokularlinse im ersten Linsenträger zu sichern.

Insbesondere bewirkt die Steuerungseinrichtung zumindest das Verschieben der Linsenträgeraufnahme entlang der zentralen Sichtachse. Die Steuerungseinrichtung kann den ersten Parameter als Grundlage für einen Steuerbefehl heranziehen, um beispielsweise das Verschieben zumindest der Linsenträgeraufnahme mit der Intraokularlinse entlang der zentralen Sichtachse veranlassen zu können. Die Linsenträgeraufnahme ist dafür mit einer Verschiebeeinheit bzw. Antriebseinrichtung verbunden, welche von einem Antrieb entlang der zentralen Sichtachse bewegt wird. Die Steuerbefehle werden von der Steuerungseinrichtung an den Antrieb gesendet, um die Linsenträgeraufnahme zu verschieben.

Alternativ oder ergänzend bewirkt die Steuerungseinrichtung zumindest das Verändern bzw. Einstellen der Brechkraft der einstellbaren Linse im optischen Strahlengang. Die Steuerungseinrichtung kann den ersten Parameter als Grundlage für einen Steuerbefehl heranziehen, um beispielsweise die Veränderung der Brechkraft veranlassen zu können. Die Steuerbefehle werden von der Steuerungseinrichtung an die einstellbare Linse gesendet, um deren Brechkraft zu verändern.

Insbesondere kann die Steuerungseinrichtung den mindestens einen Datenwert als Grundlage für einen Steuerbefehl heranziehen, um die Optiken, insbesondere die Linsenträgeraufnahme mit der Intraokularlinse, des Sehprüfsystems mithilfe von Antriebseinrichtungen entlang der zentralen Sichtachse zu verschieben. Damit ist das Einstellen der Optiken entlang der zentralen Sichtachse automatisiert möglich.

Alternativ oder ergänzend kann die Steuerungseinrichtung den mindestens einen Datenwert als Grundlage für einen Steuerbefehl heranziehen, um die Brechkraft der einstellbaren Linse im optischen Strahlengang zu verändern.

Vorzugsweise umfasst das Sehprüfsystem eine Recheneinheit, wobei der mindestens eine Datenwert in der Recheneinheit verarbeitet wird. Der mindestens eine Datenwert kann beispielweise einem Auswahlalgorithmus in der Recheneinheit zugeordnet werden, um die Auswahl der ersten Intraokularlinse für den Probanden zu vereinfachen.

Alternativ oder ergänzend wird der erste Parameter in der Recheneinheit verarbeitet. Der erste Parameter kann beispielweise einem Auswahlalgorithmus in der Recheneinheit zugeordnet werden, um die Auswahl der ersten Intraokularlinse für den Probanden zu vereinfachen. Der erste Parameter kann mit dem mindestens einen Datenwert verknüpft werden und somit die Auswahl der ersten Intraokularlinse durch den Probanden vereinfachen.

Insbesondere umfasst der Auswahlalgorithmus Rechenoperationen, welche geeignet sind, um auf Basis von gewichteten Datenwerten bzw. gewichteten mehreren Parametern die Auswahl der Intraokularlinse für den Probanden zu erleichtern.

Vorzugsweise vergleicht der Auswahlalgorithmus in der Recheneinheit zumindest einen Datenwert zur ersten Intraokularlinse mit einem weiteren Datenwert einer weiteren Intraokularlinse, welche beispielsweise ebenfalls vom Probanden im hier offenbarten Verfahren ausgewählt wurde. Damit sind unterschiedliche vom Probanden subjektiv ausgewählte Intraokularlinsen vergleichbar und der Vergleich ist dem Probanden, insbesondere an einer Anzeigeeinrichtung des Sehprüfsystem, vorzeigbar. Damit wird die Auswahl der Intraokularlinse für den Probanden vereinfacht.

Bevorzugterweise wird mindestens ein weiterer Datenwert, der während eines zweiten Sehtests mit einem Objekt repräsentativ ist, gemessen. Der Proband blickt dabei durch die zuvor ausgewählte Intraokularlinse auf zumindest einen Optotypen (Objekte bzw. Sehzeichen).

Beispielsweise umfasst der zweite Sehtest einen Kontrastsehtest, wobei die photopische Kontrasensitivität gemessen wird. Es wird nach dem gleichen Prinzip wie im Visus-Test ein Optotyp in verschiedenen Graustufen und Orientierungen resp. Abfolgen und definierter Grösse dem Probanden gezeigt. Ein kritischer Parameter ist hier der Leuchtdichteunterschied zwischen Optotyp und dem Hintergrund der Anzeigeeinrichtung, beispielsweise eine Anzeigetafel mit definierter Helligkeit. Dabei kann die Helligkeit der Darstellung eines Optotypen mit der Helligkeit des Hintergrunds der Anzeigeeinrichtung derart angepasst werden, bis die beiden Elemente nicht voneinander unterscheidbar sind. Derartige Sehtests können auch mit Anpassungen der Farbkombination bzw. des Farbkontrastes von Optotypen und der Anzeigeeinrichtung erfolgen.

Insbesondere ist die Recheneinheit ausgebildet, um Steuerbefehle für die Steuerungseinrichtung zu generieren, um ein Verschieben der Cornea-Optik und insbesondere der Linsenträgeraufnahme, zu ermöglichen. Dafür zieht die Recheneinheit einen oder mehrere hier angeführte Datenwerte beziehungsweise Parameter hinzu. Die Cornea-Optik kann an einer Antriebseinrichtung angeordnet sein, welche zumindest ein Verschieben der Cornea-Optik entlang der zentralen Sichtachse ermöglicht.

Alternativ oder ergänzend ist die Recheneinheit ausgebildet, um Steuerbefehle für die Steuerungseinrichtung zu generieren, um eine Änderung der Brechkraft der einstellbaren Linse im optischen Strahlengang herbeizuführen. Dafür zieht die Recheneinheit einen oder mehrere hier angeführte Datenwerte beziehungsweise Parameter hinzu.

Bevorzugterweise wird nach dem Schritt d) eine Kompensationslinse in die Linsenträgeraufnahme positioniert und zumindest ein Sehtest durchgeführt. Die Kompensationslinse korrigiert die optischen Komponenten im optischen Strahlengang. Die Kompensationslinse kann dabei anstelle des ersten Linsenträgers in der Linsenträgeraufnahme positioniert werden. Der Proband hat somit einen direkten Vergleich zu seinem natürlichen (unkorrigierten) Sehvermögen und dem Sehvermögen mit der ersten Intraokularlinse der vorhergehenden Überprüfung. Bei der Kompensationslinse handelt es sich in der einfachsten Form um eine sphärische oder asphärische Linse mit geeigneter Brechkraft, um die weiteren Optiken auf der zentralen Sichtachse zu korrigieren bzw. kompensieren.

Alternativ oder ergänzend wird vor dem Schritt a) eine Kompensationslinse in die Linsenträgeraufnahme positioniert und zumindest ein Sehtest durchgeführt. Der Proband hat somit einen direkten qualitativen Vergleich zu seinem natürlichen (unkorrigierten) Sehvermögen und dem Sehvermögen mit der ersten Intraokularlinse der vorhergehenden Überprüfung.

Insbesondere werden weitere Datenwerte während eines Sehtests mit der Kompensationslinse gemessen und der Recheneinheit übermittelt. Die Recheneinheit kann mithilfe eines Algorithmus die gemessenen Datenwerte mit der Kompensationslinse und die gemessen Datenwerte mit der ersten Intraokularlinse vergleichen. Der Vergleich kann dem Probanden an einer Anzeigeeinrichtung des Sehprüfsystem zur Verfügung gestellt werden.

Vorzugsweise umfasst das Sehprüfsystem zumindest eine Lichtquelle, wobei die zumindest eine Lichtquelle in den optischen Strahlengang des Sehprüfsystem während zumindest eines Sehtests eingeblendet wird. Damit kann ein Blendungstest durchgeführt werden. Im Blendungs-Test wird quantitativ die Ausdehnung des Bilds einer Lichtquelle durch eine Intraokularlinse (Halo) ermittelt. Zur Ermittlung der Grösse dieser Ausdehnung muss der Proband einen sich der Lichtquelle radial annähernden Optotyp in definierter Grösse richtig erkennen. Wird der Optotyp vom Bild der Lichtquelle, durch die von der Intraokularlinse hervorgerufenen Lichtphänomene, überstrahlt und ist somit für den Probanden nicht mehr erkennbar, ergibt sich aus dem Abstand des noch korrekt erkannten Optotyps zur Lichtquelle ein Mass für die Blendung. Dieser Datenwert kann der ersten Intraokularlinse zugeordnet werden. Die Recheneinheit kann diesen Datenwert dem Probanden derart darstellen, dass eine verbesserte Auswahl der Intraokularlinse ermöglicht ist. Auf diese Weise können Intraokularlinsen, welche für den Probanden störende oder weniger störende Lichtphänomene erzeugen, bewertet werden.

Alternativ oder ergänzend kann die zumindest eine Lichtquelle im Sehfeld des Probanden angeordnet sein, um den zuvor genannten Blendungstest mithilfe von Streulicht durchzuführen.

Bevorzugterweise ist ein Eingabegerät vorhanden, wobei der Proband das Eingabegerät während zumindest eines Sehtests bedient, um zumindest einen der gemessenen Datenwerte oder die indikative Information zuordnen zu können. Das Eingabegerät kann mit der Steuerungseinrichtung zum Austausch von Daten verbunden sein. Der Proband kann während der Sehtests selbst die für ihn ideale indikative Information oder den für ihn idealen Datenwert erfassen und der ersten Intraokularlinse zuordnen. Eine Fehlbedienung durch eine weitere Person wird ausgeschlossen. Damit ist es dem Probanden möglich, selbst die für ihn geeignete Intraokularlinse zu beurteilen. Beispielsweise erfolgt die Auswahl eines Optotyps interaktiv durch den Probanden über das Eingabegerät. Alternativ oder ergänzend erfolgt Steuerung der axialen Verschiebung der Cornea-Optik und der Linsenträgeraufnahme, während eines Sehtests durch den Proband selbst, indem er ein in die Ferne gestelltes Objekt oder eine Sehtafel für die jeweilige Augenseite durch das Sehprüfsystem mithilfe des Eingabegeräts scharfstellt. Alternativ oder ergänzend erfolgt die Änderung der Brechkraft einer einstellbaren Linse im optischen Strahlengang, während eines Sehtests durch den Proband selbst, indem er ein in die Ferne gestelltes Objekt oder eine Sehtafel für die jeweilige Augenseite durch das Sehprüfsystem mithilfe des Eingabegeräts scharfstellt.

Vorzugsweise wird zumindest eine Defokuskurve aufgenommen, um die Sehschärfe oder Kontrastsensitivität mit der ersten Intraokularlinse über den kompletten Entfernungsbereich darstellen zu können. Hierzu wird beispielsweise der zuvor angeführte Visustest in der Ferndistanz für jeden Defokuswert (Defokusposition) von -4 bis +1 Dioptrien in 0,5 Dioptrien-Schritten wiederholt. Auf diese Weise lassen sich alle Distanzen von Nah- bis Fernbereich optisch darstellen. Das Einstellen des entsprechenden Defokuswertes resp. die Verschiebung der Defokusposition erfolgt, analog zum Ausgleich der Fehlrefraktion, durch gesteuertes, axiales Verschieben einer Linse oder Linsengruppe im optischen Strahlengang, wobei das Steuern insbesondere von der Steuerungseinrichtung übernommen wird. Durch diesen semiautomatischen Ablauf ist es möglich in kürzester Zeit eine komplette Defokuskurve zu ermitteln. Alternativ oder ergänzend erfolgt die Einstellung des Defokuswertes durch Veränderung der Brechkraft einer einstellbaren Linse im optischen Strahlengang, wobei das Steuern insbesondere von der Steuerungseinrichtung übernommen wird.

Bevorzugterweise ist eine Positioniereinrichtung vorhanden, wobei zumindest der erste Linsenträger mit der ersten Intraokularlinse in die Linsenträgeraufnahme positioniert wird. Dabei kann die Positioniereinrichtung den Linsenträger je nach dessen Spezifikation positionsgenau und reproduzierbar in die Linsenträgeraufnahme positionieren. Die Positioniereinrichtung umfasst einen Antrieb, beispielsweise einen Stellantrieb, um den ersten Linsenträger zu positionieren. Fehlbedienungen durch eine weitere Person ausgeschlossen werden. Insbesondere umfasst die Positioniereinrichtung ein Magazin mit unterschiedlichen Linsenträgern und unterschiedlichen Intraokularlinsen oder Kompensationslinsen.

Insbesondere übermittelt die Steuerungseinrichtung Daten an die Positioniereinrichtung. Die Daten können Befehlsdaten oder Steuerbefehle umfassen, anhand welcher ein spezifischer Linsenträger mit einer Intraokularlinse oder einer Kompensationslinse in die Linsenträgeraufnahme positionierbar ist.

Das hierin beschriebene Zusammenwirken der Recheneinheit mit der Steuerungseinrichtung ermöglicht es den nachfolgend offenbarten mehreren Sehprüfsystemen zumindest die Auswahl der Intraokularlinsen semi-automatisch und reproduzierbar auszuführen bzw. Sehtests automatisch auszuführen. Die Fehleranfälligkeit kann damit wesentlich vermindert werden, sodass der Proband sich weitere Arztbesuche bzw. medizinische Eingriffe erspart.

Ein erfindungsgemässes Sehprüfsystem zum Überprüfen zumindest eines Seheindrucks eines Probanden mit einer Linsenträgeraufnahme zum Aufnehmen eines ersten Linsenträgers, wobei die Linsenträgeraufnahme in einer zentralen Sichtachse von distal (nahe zur betrachteten Umgebung) zu proximal (nahe zum Auge des Probanden) aufeinanderfolgend zwischen einer Cornea-Optik und einer Okular-Optik gelagert ist, umfasst eine Steuerungseinrichtung, welche ausgebildet ist zumindest ein hier vorliegend beschriebenes Verfahren zum Überprüfen zumindest eines Seheindrucks eines Probanden durchzuführen.

Das Sehprüfsystem ermöglicht es beispielsweise einem presbyopen Probanden reale Gegenstände oder Abbildungen von Gegenständen in der für das funktionelle Sehen relevanten Nah-, Intermediär- und Ferndistanz in einem ersten qualitativen Sehtest zu erfassen, um seine Wahrnehmung mit einer bestimmten, echten Intraokularlinse im Gesamtbild qualitativ in Abhängigkeit der indikativen Information zu bewerten und so die individuell beste Intraokularlinse im Sinne seiner subjektiven Beurteilung zu finden.

Insbesondere ermöglicht das Sehprüfsystem, welches eine Okular-Optik mit einer Korrekturlinse, einer Zerstreuungslinse und einer Sammellinse umfassen kann, durch die spezielle Form der Linsenoberflächen, das Sichtfeld eines durch diese blickenden Auges eines Probanden zu erweitern und gleichzeitig Defokussierungen und Kontrastfehler im erweiterten Sichtfeld zu korrigieren und bestenfalls zu eliminieren. So werden durch das erfindungsgemäße Zusammenspiel der konkav-konvexen Korrekturlinse und der nachgeschalteten Zerstreuungs- und Sammellinsen für unterschiedliche Einfallswinkel von Lichtstrahlen verschiedene Vergenzen und verschiedene Winkelunterschiede zwischen jeweiligem Eintritts- und Austrittsstrahl in Bezug auf die zentrale Sichtachse bzw. optische Achse erzeugt. Eben diese speziellen Vergenzen und Winkelunterschiede erlauben - in Kombination mit der Cornea-Optik - für unterschiedliche Sichtwinkel aus jeweiligen Raumwinkeln einfallende Lichtstrahlen im Wesentlichen auf denselben Punkt des Auges zu lenken und damit eine Sichtwinkel-Korrektur, d.h. eine fokussierte, mit der implantierten Intraokularlinse in Auflösung und Kontrast übereinstimmende Sicht durch die Intraokularlinse für unterschiedliche Sichtwinkel, zu erhalten.

Die Cornea-Optik kann dabei - in einer besonders effizienten Variante - eine durchschnittliche Hornhaut modellieren, z.B. gemäß dem Liou-Brennan-Augenmodell, um eine rasche Sichtprüfung durchzuführen; oder - in einer besonders genauen Variante - die Hornhaut des individuellen Probanden modellieren, z.B. nach deren vorheriger Vermessung, um dem individuellen Probanden eine möglichst realistische Prüfung der Intraokularlinse zu ermöglichen.

Zusammenfassend kann mithilfe der Vorrichtung der Erfindung einem Patienten schon vor der Implantation einer Intraokularlinse ein realistischer subjektiver Seheindruck - als wäre die Intraokularlinse implantiert - über ein größeres Sichtfeld gewährt und die Auswahl einer geeigneten Intraokularlinse erleichtert werden.

In einer bevorzugten Ausführungsform ist zumindest eine der einander zugewandten Linsenoberflächen der Zerstreuungs- und Sammellinsen eine Asphäre. Im Zusammenspiel mit der konkav-konvexen Korrekturlinse und der Cornea-Optik kann dadurch eine besonders gute Sichtwinkelkorrektur, d.h. eine besonders gut fokussierte, mit der implantierten Intraokularlinse für unterschiedliche Sichtwinkel in Auflösung und Kontrast übereinstimmende Sicht durch die Intraokularlinse, erzielt werden.

In einer vorteilhaften Ausführungsform ist zumindest eine der einander zugewandten Linsenoberflächen der Zerstreuungs- und Sammellinsen eine Asphäre zumindest vierter Ordnung. Die Verwendung einer solchen asphärischen Linse erlaubt, Abbildungsfehler, wie sphärische Aberrationen, Astigmatismus, etc., zu vermeiden bzw. zu korrigieren. Dadurch kann dem Probanden über ein weites Sichtfeld ein fehlerfreier Blick durch die Intraokularlinse und damit deren genaue Überprüfung ermöglicht werden. Während die Verwendung einer Asphäre höherer Ordnung eine große Flexibilität im Design der Okular-Optik bietet, ist eine Asphäre niedriger Ordnung einfacher herzustellen und deren Einfluss auf den Strahlengang einfacher vorherzusagen.

In einer günstigen Variante weist zumindest eine der Cornea- und Okular-Optiken einen Achromat oder Apochromat auf, um chromatische Aberrationen zu vermeiden. Dadurch können Linsenüberprüfungen, welche mit der implantierten Intraokularlinse in Auflösung, Fokussierung und Kontrast über ein großes Sichtfeld übereinstimmen, für eine Vielzahl an Wellenlängen durchgeführt werden. Ferner können mit einem Achromat bzw. Apochromat sphärische Aberrationen vermieden bzw. korrigiert werden. Beispielsweise kann in dieser Variante eine dispersionsfreie und vom Einfallswinkel unabhängige Kollimation eines die Vorrichtung durchlaufenden Lichtstrahls vor dem Auge des Probanden erzielt werden.

Die Cornea-Optik kann auf unterschiedliche Arten ausgeführt sein. In einer fertigungstechnisch besonders einfachen Variante weist die Cornea-Optik eine Cornea-Zerstreuungslinse und eine Cornea-Sammellinse auf, welche in beliebiger Reihenfolge entlang der optischen Sichtachse angeordnet sein können.

Wenn die Sammel- und Zerstreuungslinsen der Cornea-Optik und/oder jene der Okular-Optik unterschiedliche Abbe-Zahlen haben, bilden die jeweiligen Sammel- und Zerstreuungslinsen einen Achromat bzw. Apochromat und können so neben den sphärischen auch chromatische Aberrationen korrigieren bzw. eliminieren. Damit wird dem Probanden ermöglicht, die Intraokularlinse einer besonders genauen Überprüfung zu unterziehen und bereits vor deren Implantation über die Qualität der Intraokularlinse sowohl hinsichtlich der Schärfe als auch hinsichtlich der Farbwahrnehmung zu urteilen und so eine geeignete Intraokularlinse zu finden.

Eine verstellbare Lagerung ermöglicht eine schnelle Anpassung der Optiken des Sehprüfsystems, beispielsweise eine Verschiebung der optischen Hauptebenen einer Optik, an das gerade sichtprüfende Auge des Probanden. Dadurch kann die Vergenz des aus der Okular-Optik proximal austretenden Lichtstrahls an die Fehlsichtigkeit des Probanden angepasst werden, beispielsweise zur Korrektur sphärischer Refraktionsfehler. Durch diese Anpassung kann eine Linsenüberprüfung, welche hinsichtlich Kontrast, Auflösung und Fokussierung die Intraokularlinse im implantierten Zustand nachbildet, auch für fehlsichtige Probanden gewährleistet werden.

Alternativ oder ergänzend kann durch das Einstellen der Brechkraft einer einstellbaren Linse im optischen Strahlengang die Vergenz des aus der Okular-Optik proximal austretenden Lichtstrahls an die Fehlsichtigkeit des Probanden angepasst werden.

Ebenso ist es vorteilhaft, wenn zumindest eines der Elemente Cornea-Optik, Halterung und Okular-Optik quer zur zentralen Sichtachse auf der Trägereinrichtung verstellbar gelagert ist, um die Vorrichtung einfach justieren und kalibrieren zu können.

Das Sehprüfsystem umfasst bevorzugt eine Anzeigeeinrichtung, wie beispielsweise ein Display oder eine Sehtafel, mit der Optotypen darstellbar sind. Die Anzeigeeinrichtung kann entlang der zentralen Sichtachse verschoben werden und ergänzend dazu auch relativ zur zentralen Sichtachse verschoben werden. Dafür ist die Anzeigeeinrichtung mit einem Antrieb verbunden, der mit der Steuerungseinrichtung zum Austausch von Daten bzw. Steuerbefehlen verbunden ist. Die Recheneinheit kann zumindest einzelne der zuvor beschriebenen Daten bzw. Parameter an die Anzeigeeinrichtung senden, sodass diese dort dargestellt werden.

Das zuvor genannte Sehprüfsystem kann eine einstellbare Linse aufweisen, welche sich im optischen Strahlengang befindet und zumindest mit der Steuerungseinrichtung zum Austausch von Steuerbefehlen verbunden sein. Mithilfe der Steuerbefehle wird die Brechkraft der einstellbaren Linse verändert, sodass die Brechkraft im gesamten Sehprüfsystem einstellbar ist. In einer alternativen Ausführungsform des Sehprüfsystems ersetzt die einstellbare Linse zumindest teilweise die Antriebseinrichtungen für die Optiken der Cornea-Optik. Diese können dann an einer fixen Position an der Trägereinrichtung angeordnet sein.

Ein weiteres erfindungsgemässes Sehprüfsystem zum Überprüfen zumindest eines Seheindrucks eines Probanden umfasst eine Linsenträgeraufnahme zum Aufnehmen eines ersten Linsenträgers, wobei die Linsenträgeraufnahme in einer zentralen Sichtachse des optischen Strahlengangs von distal zu proximal aufeinanderfolgend zwischen einer Cornea-Optik und einer Okular-Optik gelagert ist. Es ist eine Intraokularlinse im Linsenträger angeordnet und eine Lichtquelle zum Einblenden von Licht in den optischen Strahlengang vorhanden. Damit kann ein Blendungstest mit dem Sehprüfsystem durchgeführt werden. Im Blendungstest wird quantitativ die Ausdehnung des Bilds einer Lichtquelle durch eine Intraokularlinse (Halo) ermittelt. Zur Ermittlung der Grösse dieser Ausdehnung muss der Proband ein sich der Lichtquelle radial annähernden Optotyp in definierter Grösse richtig erkennen. Wird der Optotyp vom Bild der Lichtquelle, durch die von der Intraokularlinse hervorgerufenen Lichtphänomene, überstrahlt und ist somit für den Probanden nicht mehr erkennbar, ergibt sich aus dem Abstand des noch korrekt erkannten Optotyps zur Lichtquelle ein Mass für die Blendung. Dieser Datenwert kann der Intraokularlinse zugeordnet werden. Eine Recheneinheit und eine Anzeigeeinrichtung, wie zuvor beschrieben, kann diesen Datenwert dem Probanden derart darstellen, dass eine verbesserte Auswahl der Intraokularlinse ermöglicht ist. Auf diese Weise können Intraokularlinsen, welche für den Probanden störende oder weniger störende Lichtphänomene erzeugen, bewertet werden, um die Auswahl der geeignetsten Intraokularlinse zu vereinfachen.

Ein erfindungsgemässer Linsenträger umfasst zumindest eine Intraokularlinse, wobei zumindest ein Datenträger am Linsenträger angeordnet ist. Am Datenträger sind Daten zu der Intraokularlinse gespeichert. Alternativ oder ergänzend sind am Datenträger Daten zum Linsenträger gespeichert.

Der Datenträger bzw. das Speichermedium kann ein codierter NFC-Chip sein, welcher beim Einlegen (elektronisch) von der Steuerungseinrichtung ausgelesen wird. Dies ermöglicht die Erkennung des Typs der Intraokularlinse samt Ablaufdatums, die Authentifizierung der Echtheit (Fälschungssicherheit) und Überprüfung der Linsenträgerdatenintegrität. So ist es möglich die Integrität im Messprozesses und der Dokumentation zu wahren und Fehlbedienungen im Untersuchungsprozess zu vermeiden indem unzulässige Augenstatus (prä- oder postoperativ) zu eingelegter Linsenträger Kombinationen erkannt werden. Die Daten können Identifikationsdaten zu der Intraokularlinse sein. Beispielsweise kann der Datenträger auch ein Barcode, ein QR-Code oder andere Speichermedien sein.

Alternativ oder ergänzend sind die Daten Kalibierdaten oder Justagedaten zu der Intraokularlinse. Des Weiteren kann anhand der auf dem NFC-Chip gespeicherten Kalibrierdaten oder Justagedaten für jeden Linsenträger vorhandene Fehlrefraktionen aufgrund der Brechkrafttoleranzen der Intraokularlinse und der Optik, sowie Fehlrefraktionen bedingt durch die Montage des Linsenträgers und des Sehprüfsystems, kompensiert werden.

Alternativ oder ergänzend sind die Daten Kalibierdaten oder Justagedaten zu dem Linsenträger. Damit ist der Linsenträger insbesondere anhand der Seriennummer einfach erkennbar oder aufgrund des Herstelldatums des Linsenträgers einfach zuzuordnen.

Die Intraokularlinsen werden dabei in einem mit flüssigkeitsgefülltem Trägermedium im Linsenträger gehalten. Beispielsweise ist der Linsenträger eine Küvette. Der Linsenträger kann magnetisch austauschbar und selbstzentrierend in der Linsenträgeraufnahme des Sehprüfsystems eingelegt werden, dafür sind Magnete und/oder Führungsschienen und/oder Führungskugeln und/oder Führungsstiften am Linsenträger angeordnet.

Ein weiteres erfindungsgemässes Sehprüfsystem zum Überprüfen zumindest eines Seheindrucks mit einer Linsenträgeraufnahme zum Aufnehmen eines ersten Linsenträgers mit einer ersten Intraokularlinse, wobei die Linsenträgeraufnahme in einer zentralen Sichtachse des optischen Strahlengangs von distal zu proximal aufeinanderfolgend zwischen einer Cornea-Optik und einer Okular-Optik gelagert ist, umfasst zumindest ein Eingabegerät, sodass mindestens ein Datenwert der ersten Intraokularlinse durch den Probanden zuordenbar ist. Der Proband kann während der Sehtests selbst die für ihn ideale indikative Information oder den für ihn idealen Datenwert erfassen und der ersten Intraokularlinse zuordnen. Eine Fehlbedienung durch eine weitere Person wird ausgeschlossen. Beispielsweise erfolgt die Erfassung eines Optotyps interaktiv durch den Probanden über das Eingabegerät. Alternativ oder ergänzend erfolgt Steuerung der axialen Verschiebung der Cornea-Optik und der Linsenträgeraufnahme, während eines Sehtests durch den Probanden selbst, indem er ein in die Ferne gestelltes Objekt oder eine Anzeigeeinrichtung, wie bspw. eine Sehtafel, für die jeweilige Augenseite durch das Sehprüfsystem scharfstellt.

Alternativ oder ergänzend erfolgt die Änderung der Brechkraft einer einstellbaren Linse im optischen Strahlengang, während eines Sehtests durch den Probanden selbst, indem er ein in die Ferne gestelltes Objekt oder eine Sehtafel für die jeweilige Augenseite durch das Sehprüfsystem mithilfe des Eingabegeräts scharfstellt.

Ein weiteres erfindungsgemässes Sehprüfsystem zum postoperativen Überprüfen zumindest eines mit einer Intraokularlinse versehenes Auges eines Probanden mit einer Linsenträgeraufnahme zum Aufnehmen eines ersten Linsenträgers, wobei die Linsenträgeraufnahme in einer zentralen Sichtachse des optischen Strahlengangs von distal zu proximal aufeinanderfolgend zwischen einer Cornea-Optik und einer Okular-Optik gelagert ist, umfasst eine Kompensationslinse zum Korrigieren der im optischen Strahlengang angeordneten Cornea-Optik und ist in der Linsenträgeraufnahme angeordnet. Die Kompensationslinse neutralisiert zumindest die angeordneten Cornea-Optik im optischen Strahlengang. Die Kompensationslinse kann dabei anstelle des ersten Linsenträgers in der Linsenträgeraufnahme positioniert werden.

Alternativ oder ergänzend ist die Kompensationslinse zum Korrigieren der im optischen Strahlengang angeordneten Okular-Optik geeignet. Die Kompensationslinse neutralisiert die angeordneten Okular-Optik im optischen Strahlengang. Die Kompensationslinse kann dabei anstelle des ersten Linsenträgers in der Linsenträgeraufnahme positioniert werden. Die Kompensation der Cornea-Optik und der Okular-Optik mit lediglich einer Kompensationslinse ist die vorteilhafte Ausführungsform, sodass diese eine Kompensationslinse die gesamte Optik des Sehprüfsystems kompensieren kann, d.h. der Proband blickt durch das Sehprüfsystem, als ob es nicht da wäre (bis auf den möglicherweise vorhandenen Refraktionsausgleich).

Postoperativ, d.h. nach Intraokularlinsen-Implantation, blickt der pseudophake Proband mit der jetzt implantierten Intraokularlinse unter den gleichen Sehbedingungen wie zuvor im Verfahren beschrieben durch das Sehprüfsystem und wiederholt die zuvor genannten Sehtests. Anstelle der Intraokularlinse (wie im prä-operativen Fall) wird die zuvor beschriebene Kompensationslinse in den optischen Strahlengang eingelegt, welche die Brechkraft der Optik im Strahlengang ausgleicht. Der Proband blickt jetzt durch seine implantierte Intraokularlinse. Prä-operative Fehlrefraktionen (Sphäre und Zylinder) sind durch die implantierte Intraokularlinse korrigiert.

Die Recheneinheit kann einen Vergleichsalgorithmus aufweisen, um die Datenwerte, welche präoperativ erfasst wurden, zumindest teilweise mit den gemessenen Datenwerten der postoperativen Überprüfung zu vergleichen. Der Vergleich der post-operativen Datenwerte mit der zuvor beschriebenen prä-operativen Messung des erreichbaren Sehens mit der Intraokularlinse erlaubt den direkten Rückschluss auf den Erfolg der Intraokularlinsen-Implantation als Mittel der Qualitätssicherung für Arzt und Probanden. Dabei kann die Recheneinheit ausgebildet sein zumindest einen Gesamtvergleichswert auszurechnen, welcher eine qualitative Aussage zum Erfolg der Intraokularlinsen-Implantation wiedergibt. Der zumindest eine Gesamtvergleichswert kann an der Anzeigeeinrichtung angezeigt werden.

Ein erfindungsgemässes Sehprüfsystem zum Aufnehmen zumindest einer Defokuskurve mit einer Linsenträgeraufnahme zum Aufnehmen eines ersten Linsenträgers, wobei die Linsenträgeraufnahme in einer zentralen Sichtachse des optischen Strahlengangs von distal zu proximal aufeinanderfolgend zwischen einer Cornea-Optik und einer Okular-Optik gelagert ist, umfasst eine Intraokularlinse, die in der Linsenträgeraufnahme angeordnet ist. Das Sehprüfsystem umfasst eine Steuerungseinrichtung und eine Recheneinheit wie zuvor beschrieben. Damit ist die Sehschärfe mit einer Intraokularlinse über den kompletten Entfernungsbereich darstellbar.

Ein erfindungsgemässes Sehprüfsystem zum Aufnehmen zumindest einer Defokuskurve mit einer Linsenträgeraufnahme zum Aufnehmen eines ersten Linsenträgers, wobei die Linsenträgeraufnahme in einer zentralen Sichtachse des optischen Strahlengangs von distal zu proximal aufeinanderfolgend zwischen einer Cornea-Optik und einer Okular-Optik gelagert ist, umfasst eine Kompensationslinse zum Korrigieren der im optischen Strahlengang angeordneten Optik und ist in der Linsenträgeraufnahme angeordnet. Das Sehprüfsystem umfasst eine Steuerungseinrichtung und eine Recheneinheit wie zuvor beschrieben. Damit ist die Sehschärfe mit einer Kompensationslinse über den kompletten Entfernungsbereich darstellbar. Hierzu ist die Steuerungseinrichtung ausgebildet einen Visustest oder Kontrastsehtest in der Ferndistanz für jeden Defokuswert (Defokusposition) von beispielsweise -4 bis +1 Dioptrien in 0,5 Dioptrien-Schritten zu wiederholen. Die Recheneinheit ist ausgebildet alle Distanzen von Nah- bis Fernbereich zu verarbeiten und an einer Anzeigeeinrichtung des Sehprüfsystems darzustellen. Das Einstellen des entsprechenden Defokuswertes resp. die Verschiebung der Defokusposition erfolgt, analog zum Ausgleich der Fehlrefraktion, durch computergesteuertes, axiales Verschieben zumindest einer der Linsen im optischen Strahlengang. Durch diesen semiautomatischen Ablauf ist es möglich in kürzester Zeit eine komplette Defokuskurve zu ermitteln. Alternativ oder ergänzend erfolgt die Einstellung des Defokuswertes durch Veränderung der Brechkraft einer einstellbaren Linse im optischen Strahlengang, wobei das Steuern insbesondere von der Steuerungseinrichtung übernommen wird.

Ein erfindungsgemässes Computerprogrammprodukt, umfassend Befehle, die bei der Ausführung des Programms durch einen Computer eines Sehprüfsystems, den Computer des Sehprüfsystems veranlassen, das Verfahren, wie zuvor offenbart auszuführen. Dabei ist das zuvor offenbarte Verfahren ein computerimplementiertes Verfahren und der Computer kann die zuvor genannte Recheneinheit sein, oder mit der Recheneinheit oder mit der Steuerungseinrichtung verknüpft sein.

Ein Erfindungsgemässes computerlesbarer Datenträger, auf dem das Computerprogrammprodukt gespeichert ist.

Weitere Vorteile, Merkmale und Einzelheiten der Erfindung ergeben sich aus der nachfolgenden Beschreibung, in der unter Bezugnahme auf die Zeichnungen Ausführungsbeispiele der Erfindung beschrieben sind.

Die Bezugszeichenliste ist wie auch der technische Inhalt der Patentansprüche und Figuren Bestandteil der Offenbarung. Die Figuren werden zusammenhängend und übergreifend beschrieben. Gleiche Bezugszeichen bedeuten gleiche Bauteile, Bezugszeichen mit unterschiedlichen Indices geben funktionsgleiche oder ähnliche Bauteile an.

Mittels der nachfolgenden Figuren wird anhand von Ausführungsbeispielen die Erfindung näher erläutert.

Positionsangaben, wie "oben", unten", "rechts" oder "links" sind jeweils auf die entsprechenden Darstellungen bezogen und sind nicht als einschränkend zu verstehen.

Obwohl die Erfindung mittels der Figuren und der zugehörigen Beschreibung dargestellt und detailliert beschrieben ist, sind diese Darstellung und diese detaillierte Beschreibung illustrativ und beispielhaft zu verstehen und nicht als die Erfindung einschränkend. Es versteht sich, dass Fachleute Änderungen und Abwandlungen machen können, ohne den Umfang der folgenden Ansprüche zu verlassen. Insbesondere umfasst die Erfindung ebenfalls Ausführungsformen mit jeglicher Kombination von Merkmalen, die vorstehend zu verschiedenen Aspekten und/oder Ausführungsformen genannt oder gezeigt sind.

Die Erfindung umfasst ebenfalls einzelne Merkmale in den Figuren, auch wenn sie dort im Zusammenhang mit anderen Merkmalen gezeigt sind und/oder vorstehend nicht genannt sind. Im Weiteren schliesst der Ausdruck "umfassen" und Ableitungen davon andere Elemente oder Schritte nicht aus. Ebenfalls schliesst der unbestimmte Artikel "ein" beziehungsweise "eine" und Ableitungen davon eine Vielzahl nicht aus. Die Funktionen mehrerer in den Ansprüchen aufgeführter Merkmale können durch eine Einheit erfüllt sein. Die Begriffe "im Wesentlichen", "etwa", "ungefähr" und dergleichen in Verbindung mit einer Eigenschaft beziehungsweise einem Wert definieren insbesondere auch genau die Eigenschaft beziehungsweise genau den Wert. Alle Bezugszeichen in den Ansprüchen sind nicht als den Umfang der Ansprüche einschränkend zu verstehen.

### Figurenbeschreibung

Die Figuren werden zusammenhängend und übergreifend beschrieben. Gleiche Bezugszeichen bedeuten gleiche Bauteile. Es zeigen
Fig. 1 eine erste Ausführungsform eines erfindungsgemässen Sehprüfsystems zum Überprüfen zumindest eines Seheindrucks in einer schematischen Darstellung,
Fig. 2: eine erste Optik-Einheit (Cornea-Optik) des Sehprüfsystems gemäss Fig. 1 in einer schematischen Darstellung,
Fig. 3: eine weitere Optik-Einheit (Okular-Optik) des Sehprüfsystems gemäss Fig. 1 in einer schematischen Darstellung,
Fig. 4 : eine erste Ausführungsform eines erfindungsgemässen Verfahrens zum präoperativen Überprüfen des Seheindrucks in einem Ablaufdiagramm,
Fig. 5 : das Verfahren gemäss Fig. 4 und zusätzlich Schritte zum postoperativen Überprüfen des Seheindrucks in einem Ablaufdiagramm,
Fig. 6 : eine schematische Darstellung zur prä-operativen Beurteilung des subjektiven Seheindrucks mit einer Intraokularlinse,
Fig. 7 : eine schematische Darstellung zur prä-operativen Beurteilung des subjektiven Seheindrucks mit einer Kompensationslinse,
Fig. 8: eine schematische Darstellung zur prä-operativen Beurteilung des subjektiven Seheindrucks zur Blendungsempfindlichkeit mit einer Intraokularlinse;
Fig. 9: eine schematische Darstellung zur prä-operativen Messung des erreichbaren Visus und Kontrastsehens durch eine Intraokularlinse im Nah-, Intermediär- und Fernbereich,
Fig. 10: eine schematische Darstellung zur prä-operativen Messung von Dysphotopsien einer in der Ferndistanz befindlichen Lichtquelle durch eine Intraokularlinse,
Fig. 11: eine schematische Darstellung zur prä-operativen Aufnahme einer Defokuskurve durch eine Intraokularlinse,
Fig. 12: eine schematische Darstellung zur post-operativen Messung des erreichten Visus und Kontrastsehens durch eine im Auge implantierte Intraokularlinse im Nah-, Intermediär- und Fernbereich,
Fig. 13: eine schematische Darstellung zur post-operativen Messung von Dysphotopsien einer in der Ferndistanz befindlichen Lichtquelle durch die implantierte Intraokularlinse,
Fig. 14: eine schematische Darstellung zur post-operativen Aufnahme einer Defokuskurve durch die implantierte Intraokularlinse,
Fig. 15: einen erfindungsgemässen Linsenträger mit einer Intraokularlinse,
Fig. 16: eine weitere Ausführungsform eines erfindungsgemässen Sehprüfsystems zum Überprüfen zumindest eines Seheindrucks in einer schematischen Darstellung,
Fig. 17: eine weitere Ausführungsform eines erfindungsgemässen Sehprüfsystems zum Überprüfen zumindest eines Seheindrucks in einer schematischen Darstellung, und
Fig. 18: eine weitere Ausführungsform eines erfindungsgemässen Sehprüfsystems zum Überprüfen zumindest eines Seheindrucks in einer schematischen Darstellung.

### Ausführung der Erfindung

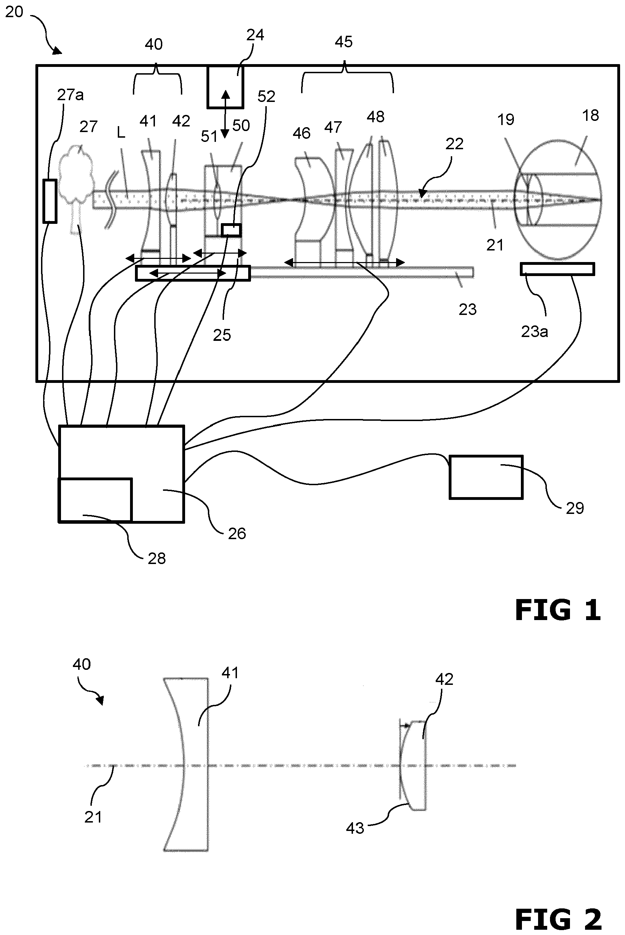

**Figur 1** bis **Figur 3** zeigt eine erste Ausführungsform eines Sehprüfsystems 20 umfassend eine Trägereinrichtung 23 auf der eine Linsenträgeraufnahme 25 mit einem Linsenträger 50 samt Intraokularlinse 51 angeordnet ist. Die Linsenträgeraufnahme 25 ist in einer zentralen Sichtachse 21 des optischen Strahlengangs 22 von distal (nahe zur betrachteten Umgebung) zu proximal (nahe zum Auge des Probanden 18) aufeinanderfolgend zwischen einer Cornea-Optik 40 und einer Okular-Optik 45 gelagert. Die Trägereinrichtung 23 kann dafür jede in der Optik bekannte Einrichtung zum Lagern optischer Elemente sein, z.B. ein Rahmen, ein Gehäuse, ein Rohr, eine optische Bank, ein optischer Tisch, usw. Die Cornea-Optik 40 (oder Teile davon), die Linsenträgeraufnahme 25 (oder Teile davon) und/oder die Okular-Optik 45 (oder Teile davon) sind entlang der zentralen Sichtachse 21 verschieblich und/oder normal dazu verstellbar an der Trägereinrichtung 23 gelagert, um sie relativ zueinander zu justieren. Die zuvor genannten Optiken weisen dafür Antriebseinrichtungen mit Antrieben (als horizontale Doppelpfeile dargestellt) auf, welche das Verschieben der Optiken ermöglichen.

Der Aufbau des Sehprüfsystems 20 soll im Folgenden bei gerader Durchsicht entlang der zentralen Sichtachse 21 eines repräsentativer Lichtstrahls L beschrieben werden. Zuerst passiert ein von der Umgebung bzw. von der Seite einer Anzeigeeinrichtung 27, d.h. vom distalen Ende des Sehprüfsystems 20 her, einfallender Lichtstrahl L die Cornea-Optik 40. Die Cornea-Optik umfasst eine distale Cornea-Zerstreuungslinse 41 und eine proximale Cornea-Sammellinse 42. **Figur 2** zeigt die Cornea-Optik 40 im Detail. Die distale Linsenoberfläche 43 der Cornea-Sammellinse 42 ist eine Asphäre. In einer Variante ist diese Asphäre vierter Ordnung.

Nach der Cornea-Optik 40 wird der Lichtstrahl L durch die Intraokularlinse 51 der Linsenträgeraufnahme 25 geführt, wo sie - so als ob sie im Auge des Probanden 18 implantiert wäre - den Lichtstrahl L ihrer Brechkraft gemäß fokussiert.

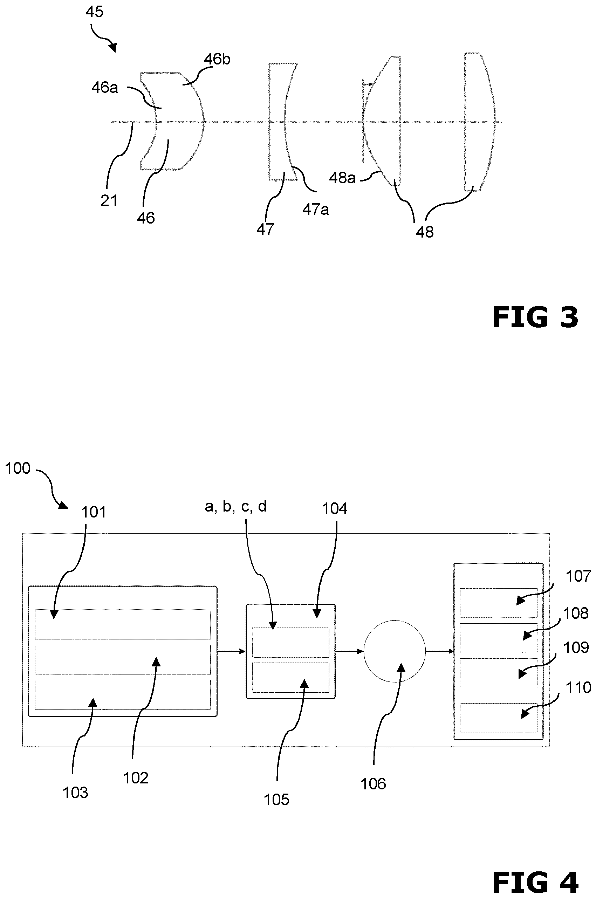

Anschliessend durchquert der Lichtstrahl L die Okular-Optik 45, welche eine distale Korrekturlinse 46 und, proximal dazu, eine Zerstreuungslinse 47 und eine Sammellinse 48 umfasst und trifft auf die Iris 19 des Probanden 18. Alternativ zur gezeigten Ausführungsform kann die Sammellinse 48 distal zur Zerstreuungslinse 47 angeordnet sein. Die Korrekturlinse 46 hat eine distale konkave Linsenoberfläche 46a und eine proximale konvexe Linsenoberfläche 46b, siehe **Figur 3****.** Die Zerstreuungslinse 47 ist planar-konkav und die Sammellinse 48 umfasst ein erstes konvexplanares Segment und ein zweites planar-konvexes Segment. Dadurch ist die der asphärischen Linsenoberfläche 48a gegenüberliegende Linsenoberfläche planar und die Asphäre einfacher herzustellen. Optional ist zumindest eine oder beide der einander zugewandten Linsenoberflächen 47a, 48a der Zerstreuungs- und Sammellinsen 47, 48 eine Asphäre (hier: die distale Linsenoberfläche 48a der Sammellinse 48).

Das Sehprüfsystem 20 umfasst eine Steuerungseinrichtung 26 und der erste Linsenträger 50 ein Speichermedium 52 bzw. einen Datenträger, wobei ein erster Parameter mithilfe von Datenleitungen bzw. kabellosen Datenverbindungen von dem Speichermedium 52 des ersten Linsenträgers 50 an die Steuerungseinrichtung 26 übermittelbar ist, wenn der erste Linsenträger 50 in die Linsenträgeraufnahme 25 positioniert wird. Die Steuerungseinrichtung 26 zieht den ersten Parameter als Grundlage für einen Steuerbefehl heran, um den Linsenträger 50 zu erkennen. Handelt es sich um einen invaliden Linsenträger 50 so wird ein Warnsignal an die Anzeigeeinrichtung 27 des Sehprüfsystems 20 ausgeben. Dabei umfasst der erste Parameter zumindest eine der folgenden Informationen, nämlich Parameter zur Echtheit bzw. Validität des Linsenträgers (Zertifikat), Name, Artikelnummer, Herstelldatum, Haltbarkeit, Seriennummer und Position im Strahlengang, Justagewerte, Kalibrierwerte und einen Fokuspunkt in Abhängigkeit von der Addition in der ersten Intraokularlinse 51. Die Steuereinrichtung ist mithilfe von Datenleitungen mit den Antriebseinrichtungen der Optiken verbunden, um Daten zu empfangen und Steuerbefehle zum Antreiben der Antriebe, und damit zum Verschieben der Optiken, zu übermitteln.

Die Steuerungseinrichtung 26 ist ausgebildet, um zumindest das Verschieben der Linsenträgeraufnahme 25 entlang der zentralen Sichtachse 21 zu bewirken. Die Steuerungseinrichtung nimmt einen oder mehrere der vorgenannten Parameter als Grundlage für einen Steuerbefehl, um das Verschieben zumindest der Linsenträgeraufnahme 25 mit der Intraokularlinse 51 entlang der zentralen Sichtachse 21 zu veranlassen. Die Linsenträgeraufnahme 25 ist dafür mit einer Verschiebeeinheit (Doppelpfeil) verbunden, welche von einem Antrieb entlang der zentralen Sichtachse 21 bewegt wird. Die Steuerbefehle werden von der Steuerungseinrichtung 26 an den Antrieb gesendet, um die Linsenträgeraufnahme 25 zu verschieben. Des Weiteren ist die Steuerungseinrichtung 26 ausgebildet, um zumindest das Verschieben der Cornea-Optik 40 und der Okular-Optik 45 entlang der zentralen Sichtachse 21 zu bewirken. Das Sehprüfsystem 20 weist eine Recheneinheit 28 auf. Die Recheneinheit 28 kann ein Computer bzw. ein Microprozessor sein und ist mit der Steuerungseinrichtung 26 verbunden. Die Recheneinheit 28 ist ausgebildet, um Steuerbefehle für die Steuerungseinrichtung 26 zu generieren, um ein Verschieben der Optiken 40, 45 und der Linsenträgeraufnahme 25 zu ermöglichen.

Des Weiteren ist ein Eingabegerät 29 vorhanden, wobei der Proband 18 das Eingabegerät 29 während zumindest eines Sehtests bedient, um zumindest einen gemessenen Datenwerte oder eine indikative Information der Intraokularlinse 51 zuordnen zu können. Das Eingabegerät 29 ist mit der Steuerungseinrichtung 26 zum Austausch von Daten verbunden. Mithilfe des Eingabegeräts 29 ist es dem Probanden 18 möglich, aktuelle Positionen der Intraokularlinse 51 bzw. der Optiken 40, 45 auf der Trägereinrichtung 23 zu bestätigen und damit ein spezielles Setup der Komponenten auf der Trägereinrichtung 23 in der Recheneinheit 28 bzw. der Steuerungseinrichtung 26 zu hinterlegen.

Das Sehprüfsystem 20 weist eine Positioniereinrichtung 24, beispielsweise ein Wechselsystem oder Revolvermagazin, auf, wobei zumindest der erste Linsenträger 50 mit der ersten Intraokularlinse 51 in der Linsenträgeraufnahme 25 positioniert wird. Dabei kann die Positioniereinrichtung 24 den Linsenträger 50 je nach dessen Spezifikation positionsgenau und reproduzierbar in die Linsenträgeraufnahme 25 setzen. Die Positioniereinrichtung 24 umfasst einen Antrieb (Doppelpfeil), beispielsweise einen Stellantrieb, um den ersten Linsenträger 25 zu positionieren und ist zum Austausch von Daten und Steuerbefehlen mit der Steuerungseinrichtung 26 verbunden.

Die Anzeigeeinrichtung 27 ist ein Display oder eine Sehtafel, mit der Optotypen darstellbar sind. Die Anzeigeeinrichtung 27 kann entlang der zentralen Sichtachse 21 verschoben werden und ergänzend dazu auch relativ zur zentralen Sichtachse 21 verschoben werden. Dafür ist die Anzeigeeinrichtung mit einem Antrieb verbunden, der mit der Steuerungseinrichtung 26 zum Austausch von Daten bzw. Steuerbefehlen verbunden ist. Die Recheneinheit 28 kann zumindest einzelne der zuvor beschriebenen Daten bzw. Parameter zu Sehtests oder von der Recheneinheit 28 analysierte Daten an die Anzeigeeinrichtung 27 senden, sodass diese dort dargestellt werden.

Das Sehprüfsystem 20 umfasst eine Lichtquelle 27a, wobei die Lichtquelle 27a in den optischen Strahlengang 22 des Sehprüfsystems 20 während zumindest eines Sehtests eingeblendet wird. Die Lichtquelle 27a ist mit der Steuerungseinrichtung 26 zum Austausch von Daten und Steuerbefehlen mittels Datenkabel verbunden. Alternativ kann die Lichtquelle 27a mithilfe einer optischen Einheit, ein Strahlteiler oder ein Spiegel, in den optischen Strahlengang 22 eingeblendet werden.

Das Sehprüfsystem 20 umfasst eine höhenverstellbare Kinn- oder Kopfstütze 23a zur vertikalen Positionierung des Kopfes des Probanden 18. Die höhenverstellbare Kinn- oder Kopfstütze 23a ist mit der Steuerungseinrichtung 26 zum Austausch von Daten und Steuerbefehlen mittels Datenkabel verbunden.

In der Recheneinheit 28 werden Befehle eines Computerprogrammprodukts derart abgearbeitet, sodass das nachfolgend beschriebene Verfahren zum Überprüfen zumindest eines Seheindrucks eines Probanden auf einem Sehprüfsystem ausführbar ist.

**Figur 4** zeigt eine erste Ausführungsform eines erfindungsgemässen Verfahrens 100 zum Überprüfen zumindest eines Seheindrucks eines Probanden 18, welches mit einem Sehprüfsystem 20 gemäss der **Figur 1** bis **Figur 3** ausführbar ist. Das Verfahren 100 umfasst zumindest die folgenden Schritte:
a. Positionieren eines ersten Linsenträgers 50 mit einer ersten Intraokularlinse 51 in der Linsenträgeraufnahme 25 des Sehprüfsystems 20;
b. Verschieben der Cornea-Optik 40 und der Linsenträgeraufnahme 25 mit der Intraokularlinse 51 entlang der zentralen Sichtachse 21, wobei die Okular-Optik 45 in einem fixen Abstand zu zumindest einem Auge des Probanden 18 verbleibt;
c. Erfassen zumindest einer für den Seheindruck indikativen Information;
d. Zuordnen der mindestens einen indikativen Information zur ersten Intraokularlinse 51.

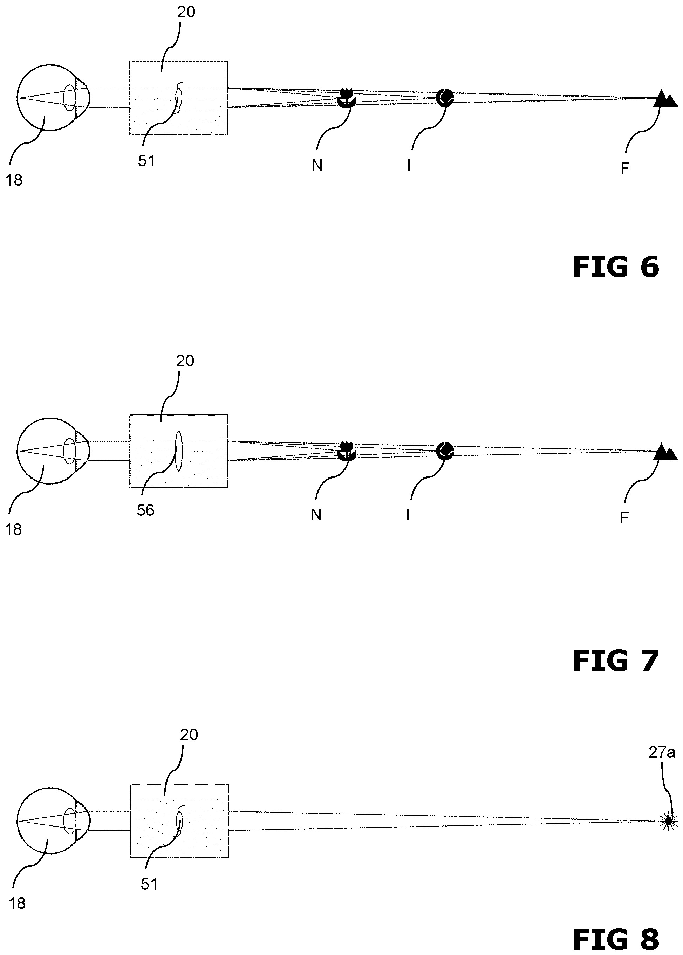

Das Verfahren ermöglicht es einem Probanden reale Gegenstände oder Optotypen, welche entlang der zentralen Sichtachse 21 angeordnet sind in einem ersten qualitativen Sehtest zu erfassen (Schritt 104), um seine Wahrnehmung mit der in Linsenträgeraufnahme 25 positionierten echten Intraokularlinse 51 im Gesamtbild qualitativ in Abhängigkeit der indikativen Information zu bewerten und so die individuell beste Intraokularlinse 51 im Sinne seiner subjektiven Beurteilung zu finden. Zusätzlich kann mithilfe der Kompensationslinse 56 ein Vergleich mit dem natürlichen (unkorrigierten) Sehen erfolgen. Die indikative Information umfasst nicht abschliessend aufgezählt zumindest einen der nachfolgenden Parameter, nämlich Kontrast, Schärfe, Farbe, Streulichteffekte, Lichtsensation, oder Dysphotopsie. Der Proband 18 blickt dabei durch die Intraokularlinse 51 und es werden ihm Objekte oder Optotypen an der Anzeigeeinrichtung 27 des Sehprüfsystems 20 oder reale Gegenstände im Nah-, Intermediär- und Fernbereich N, I, F angezeigt - **siehe** **Figur 6****.** Abhängig von der durch den Probanden 18 subjektiv wahrgenommen indikativen Information, erfolgt schlussendlich eine subjektive Beurteilung der Intraokularlinse 51.

Im Schritt a. wird mindestens ein erster Parameter mithilfe von Datenleitungen von dem Speichermedium 52 des ersten Linsenträgers 50 an die Steuerungseinrichtung 26 übermittelbar. Dabei umfasst der erste Parameter beispielsweise die Echtheit bzw. Validität des Linsenträgers (Zertifikat) oder eine Seriennummer oder eine Bezeichnung der Intraokularlinse 51. Der erste Parameter wird in der Recheneinheit 28 verarbeitet. Der erste Parameter kann beispielweise einem Auswahlalgorithmus in der Recheneinheit 28 zugeordnet werden, um die Auswahl der ersten Intraokularlinse 51 für den Probanden 18 zu vereinfachen.

Nach dem Schritt d) kann der Proband eine Kompensationslinse 56 in die Linsenträgeraufnahme 25 positionieren und zumindest einen Sehtest durchführen. Die Kompensationslinse 56 kann dabei anstelle des ersten Linsenträgers 50 in der Linsenträgeraufnahme 25 positioniert werden.

Der Proband 18 blickt dabei durch die Kompensationslinse 56 und es werden ihm Objekte oder Optotypen an der Anzeigeeinrichtung 27 des Sehprüfsystems 20, oder reale Gegenstände im Nah-, Intermediär- und Fernbereich N, I, F angezeigt - **siehe** **Figur 7****.**

Alternativ oder ergänzend wird vor dem Schritt a) eine Kompensationslinse in die Linsenträgeraufnahme positioniert und zumindest ein Sehtest durchgeführt. Der Proband hat somit einen direkten qualitativen Vergleich zu seinem natürlichen (unkorrigierten) Sehvermögen und dem Sehvermögen mit der ersten Intraokularlinse der vorhergehenden Überprüfung.

Wird die erste Intraokularlinse 51 vom Probanden 18 als ungeeignet bewertet, werden die zuvor genannten Verfahrensschritte wiederholt ausgeführt, wobei im Schritt a. ein zweiter Linsenträger mit einer zweiten Intraokularlinse anstelle des ersten Linsenträgers 50 in der Linsenträgeraufnahme 25 des Sehprüfsystems 20 positioniert wird. Sollte auch die zweite Intraokularlinse vom Probanden 18 als ungeeignet empfunden werden, so kann der Linsenträger mit der zweiten Intraokularlinse durch einen weiteren Linsenträger mit einer weiteren Intraokularlinse getauscht werden und das zuvor genannte Verfahren wiederholt werden.

Vor dem Schritt a. erfolgt eine Probandenjustage 101, bei der der Proband 18 korrekt auf das Sehprüfsystem 20 eingestellt wird, sodass zumindest ein Auge des Probanden 18 auf der zentralen Sichtachse 21 des optischen Strahlengangs 22 zentriert ist. Dies erfolgt einerseits durch eine höhenverstellbare Kinn- oder Kopfstütze 23 zur vertikalen Positionierung des Probanden 18 und andererseits durch Verstellen des Abstands zumindest eines optischen Strahlengangs 22 im Sehprüfsystem 20 auf die Pupillendistanz der Augen des Probanden 18. Alternativ oder zusätzlich dazu kann auch die Position der Optik vertikal auf die Position des Auges des Probanden eingestellt werden. In einem weiteren Schritt 102 wird ein eventuell vorhandener regulärer Astigmatismus im Auge des Probanden 18 durch zylindrische Vorsatzlinsen oder einer Stokes-Linse kompensiert. Die zylindrische Vorsatzlinsen (oder Stokes-Linse) können in der entsprechenden Stärke und Achslage in den optischen Strahlengang 22 des Sehprüfsystems 20 für die jeweilige Augenseite positioniert werden. Die Stokes-Linse kann auch in das Gerät integriert sein.

Zum Einstellen einer eventuell vorhandenen sphärischen Fehlrefraktion des Auges des Probanden 18, wird durch axiales Verschieben der Cornea-Optik 40 im optischen Strahlengang 22 die Brechkraft des gesamten optischen Systems verändert (Schritt 103).

In einem weiteren Schritt 105 kann ein qualitativer Blendungstest durchgeführt -siehe auch **Figur 8****.** Der Proband 18 blickt dabei wieder durch die in der Linsenträgeraufnahme 25 angeordnete Intraokularlinse 51. Für diesen Sehtest umfasst das Sehprüfsystem 20 zumindest eine Lichtquelle 24, wobei die zumindest eine Lichtquelle 24 in den optischen Strahlengang 22 des Sehprüfsystems 20 während zumindest eines Sehtests eingeblendet wird. Im Blendungstest wird qualitativ die Ausdehnung des Bilds der Lichtquelle 24 (Halo) durch die Intraokularlinse 51 bewerten.

In einer nicht gezeigten Ausführungsform ist die zumindest eine Lichtquelle 24 im Sehfeld des Probanden 18 angeordnet, um den zuvor genannten Blendungstest mithilfe von Streulicht durchzuführen.

Mithilfe der vorhergenannten Schritte 101-105 wählt der Proband 18 eine spezifischen Intraokularlinse 51 aus (Schritt 106).

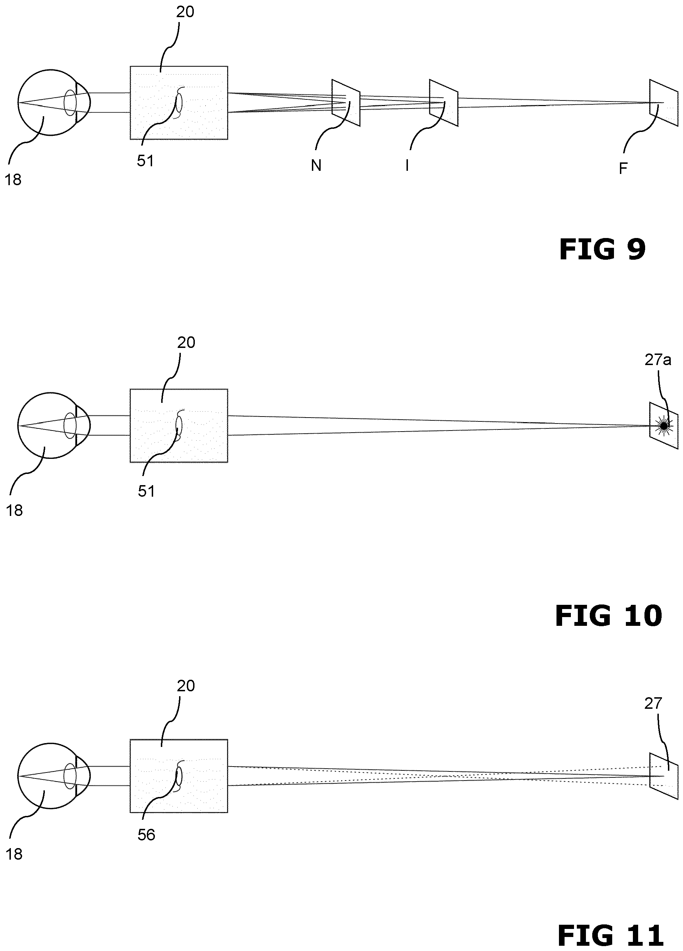

In einem nächsten Schritt 107 führt der Proband 18 einen ersten quantitativen Sehtest durch. Der Proband 18 blickt dabei durch die zuvor ausgewählte Intraokularlinse 51 auf zumindest einen Optotypen (Objekte bzw. Sehzeichen) der Anzeigeeinrichtung 27. Beispielsweise ist der erste Sehtest einen Visustest, bei dem Optotypen in verschieden Grössen und Orientierungen resp. Abfolgen an der Anzeigeeinrichtung 27 des Sehprüfsystems 20 im Nah-, Intermediär- und Fernbereich N, I, F angezeigt werden. Dabei werden Sehschärfewerte als Datenwerte vom durch die Intraokularlinse 51 blickenden Probanden gemessen - siehe **Figur 9****.**

Die gemessenen Sehschärfewerte werden an die Steuerungseinrichtung 26 bzw. der Recheneinheit 28 weitergeleitet und in der Recheneinheit 28 verarbeitet. Mindestens ein Sehschärfewert wird in einem Auswahlalgorithmus in der Recheneinheit 28 weiterverarbeitet. Dabei können beispielsweise verschieden Grössen und Orientierungen resp. Abfolgen der Optotypen, oder ein ermittelter Sehschärfewert, der ersten Intraokularlinse 51 zugeordnet werden. Werden mehrere Datenwerte erfasst, entsteht ein Datensatz von Datenwerten, welche die vorliegend getestete erste Intraokularlinse 51 für den Probanden 18 charakterisiert und mit dem Auge des Probanden 18 verlinkt. Der Datensatz kann bei der schlussendlichen Auswahl der Intraokularlinse 51 für eine Auswertung herangezogen werden.

Die Steuerungseinrichtung 26 ist ausgebildet mindestens einen Datenwert bzw. mindestens einen Sehschärfewert als Grundlage für einen Steuerbefehl heranziehen, um die Optiken des Sehprüfsystems 20 auf den Probanden 18 und der ersten Intraokularlinse 51 einzustellen. Das Zusammenwirken der Steuerungseinrichtung 26 mit der Recheneinheit 28 ermöglicht eine automatische Einstellung von Positionen der Optiken 40, 45 und des Linsenträgers 50 auf der Trägereinrichtung 23.

In einem nächsten Schritt 108 führt der Proband 18 einen weiteren Sehtest durch. Der Proband 18 blickt dabei durch die zuvor ausgewählte Intraokularlinse 51 auf zumindest einen Optotypen (Objekte bzw. Sehzeichen). Der weitere Sehtest ist ein Kontrastsehtest, wobei die photopische Kontrasensitivität gemessen wird. Es wird nach dem gleichen Prinzip wie im Visustest ein Optotyp in verschiedenen Graustufen und Orientierungen resp. Abfolgen und definierter Grösse an der Anzeigeeinrichtung 27 des Sehprüfsystems 20 im Nah-, Intermediär- und Fernbereich N, I, F dem Probanden 18 gezeigt. Dabei werden Datenwerte zur photopische Kontrasensitivität vom durch die Intraokularlinse 51 blickenden Probanden gemessen.

In einem weiteren Schritt 109 wird ein Blendungstest durchgeführt - siehe auch **Figur 10****.** Im Blendungstest wird quantitativ die Ausdehnung des Bilds der Lichtquelle 24 durch eine Intraokularlinse 51 ermittelt. Zur Ermittlung der Größe dieser Ausdehnung muss der Proband 18 ein sich der Lichtquelle 27a radial annähernden Optotyp in definierter Größe richtig erkennen. Wird der Optotyp vom Bild der Lichtquelle 27a, durch die von der Intraokularlinse 51 hervorgerufenen Lichtphänomene, überstrahlt und ist somit für den Probanden 51 nicht mehr erkennbar, ergibt sich aus dem Abstand des noch korrekt erkannten Optotyps zur Lichtquelle 27a ein Maß für die Blendung. Der gemessene Abstand wird als Datenwert erfasst und der Intraokularlinse 51 zugeordnet.

Alternativ oder ergänzend kann die zumindest eine Lichtquelle im Sehfeld des Probanden angeordnet sein, um den zuvor genannten Blendungstest mithilfe von Streulicht durchzuführen.

In einem weiteren Schritt 110 kann zumindest eine Defokuskurve aufgenommen, um die Sehschärfe oder Kontrastsensitivität mit der ersten Intraokularlinse 51 über den kompletten Sehentfernungsbereich darstellen zu können - siehe **Figur 11****.** Hierzu wird beispielsweise der zuvor angeführte Visustest in der Ferndistanz für jeden Defokuswert (Defokusposition) von -4 bis +1 Dioptrien in 0,5 Dioptrien-Schritten wiederholt. Auf diese Weise lassen sich alle Distanzen von Nah- bis Fernbereich optisch darstellen. Das Einstellen des entsprechenden Defokuswertes resp. die Verschiebung der Defokusposition erfolgt, analog zum Ausgleich der Fehlrefraktion, durch gesteuertes, axiales Verschieben einer Linse oder Linsengruppe im optischen Strahlengang 22, wobei das Steuern insbesondere von der Steuerungseinrichtung 26 übernommen wird.

Die Steuerungseinrichtung 26 ist ausgebildet die gemessen Datenwert als Grundlage für einen Steuerbefehl heranziehen, um die Optiken 40, 45 des Sehprüfsystems 20 auf den Probeanten 18 und der ersten Intraokularlinse 51 einzustellen.

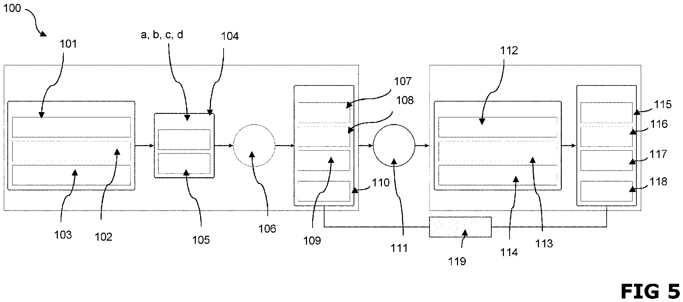

**Figur 5** zeigt das zuvor in **Figur 4** offenbarte Verfahren, wobei nach der Auswahl der Intraokularlinse 51 durch den Probanden 18 eine Implantation 111 der Intraokularlinse 51 durch einen operativen Eingriff in das Auge des Probanden 18 erfolgt. Der chirurgische Eingriff ist nicht Teil des erfindungsgemässen Verfahrens. Jedoch kann der Proband 18 das zuvor in **Figur 1** bis **Figur 3** offenbarte Sehprüfsystem dazu verwenden, einen postoperative Überprüfung des Sehvermögens mit der zuvor ausgewählten Intraokularlinse 51, welche sich nun in seinem Auge befindet zu testen. Während des nachfolgend beschriebenen Verfahrens, befindet sich ein Linsenträger 55 mit einer Kompensationslinse 56 in der Linsenträgeraufnahme 25, welche die Optiken im optischen Strahlengang 22 ausgleicht bzw. kompensiert.

Bei diesem Verfahren wird wiederum zuerst eine Probandenjustage 112 durchgeführt, bei der der Proband 18 korrekt auf das Sehprüfsystem 20 eingestellt wird, so dass zumindest ein Auge des Probanden 18 auf der zentralen Sichtachse 21 des jeweiligen Strahlengangs zentriert ist, analog zum Schritt 101. In einem weiteren Schritt 113 kann ein eventuell vorhandener regulärer Astigmatismus im Auge des Probanden 18 durch zylindrische Vorsatzlinsen oder einer Stokes-Linse kompensiert werden - analog zum Schritt 102.

Zum Einstellen einer eventuell vorhandenen sphärischen Fehlrefraktion des Auges des Probanden, ist durch axiales Verschieben einer Optik (Linse oder Linsengruppe), insbesondere der Cornea-Optik 40, im optischen Strahlengang 22 die Brechkraft des gesamten optischen Systems verändert werden (Schritt 114).

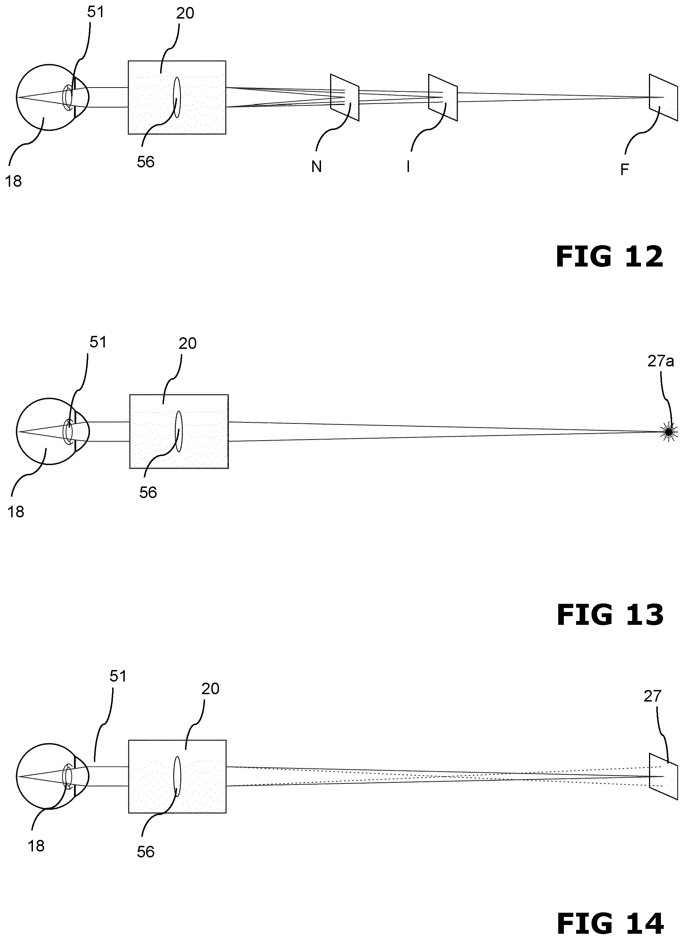

Postoperativ, d.h. nach Intraokularlinsen-Implantation, blickt der pseudophake Proband 18 mit der jetzt implantierten Intraokularlinse 51 unter den gleichen Sehbedingungen, wie zuvor im Verfahren gemäss **Figur 4** beschrieben, durch das Sehprüfsystem 20 und wiederholt die zuvor genannten Sehtests gemäss den Schritten 107-110 (nun die Schritte 115 - 118). Anstelle der Intraokularlinse 51 (wie im prä-operativen Fall) wird die zuvor beschriebene Kompensationslinse 56 in den optischen Strahlengang 22 eingelegt- siehe **Figur 12** bis **Figur 14****.**

Zu jedem Sehtest der Schritte 115 - 118 wird jeweils mindestens ein Datenwert gemessen und der Steuereinrichtung 26 bzw. der Recheneinheit 28 übermittelt. Die Recheneinheit 28 weist einen Vergleichsalgorithmus auf, um die Datenwerte, welche präoperativ in den Schritten 107-110 erfasst wurden, zumindest teilweise mit den gemessenen Datenwerten der Schritte 115 - 118 zu vergleichen. Der Vergleich 119 der post-operativen Datenwerte mit der zuvor beschriebenen prä-operativen Messung des erreichbaren Sehens mit einer Intraokularlinse 51 erlaubt den direkten Rückschluss auf den Erfolg der Intraokularlinsen-Implantation als Mittel der Qualitätssicherung für Arzt und Probanden 18. Dabei ist die Recheneinheit 28 ausgebildet zumindest einen Gesamtvergleichswert auszurechnen, welcher eine qualitative Aussage zum Erfolg der Intraokularlinsen-Implantation wiedergibt.

Die zuvor genannten Schritte können als Befehle in einem Computerprogrammprodukt gespeichert sein.

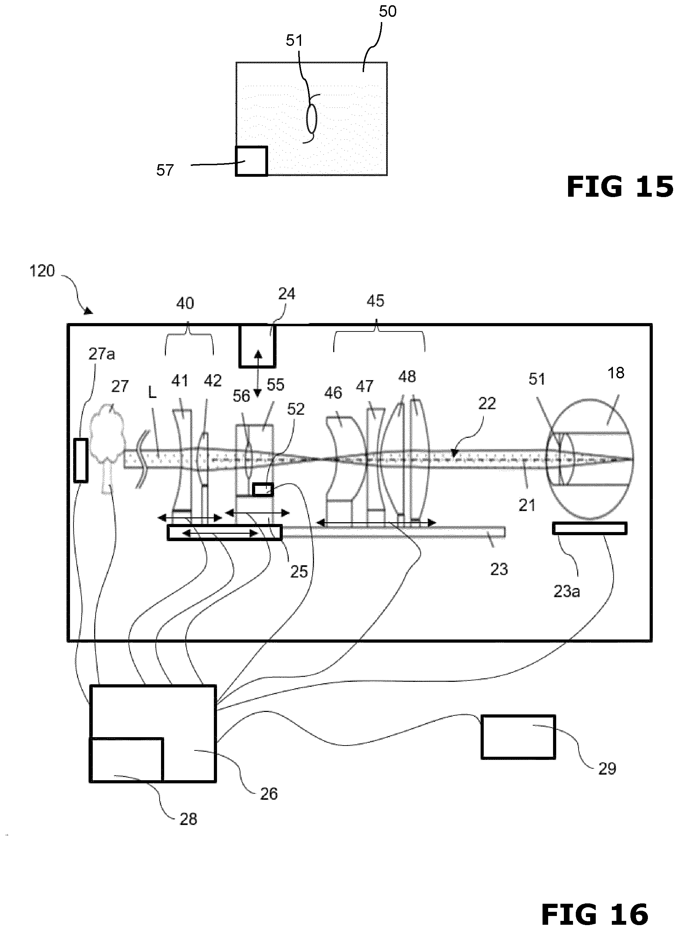

**Figur 15** zeigt eine erste Ausführungsform des Linsenträgers 50 mit einer Intraokularlinse 51, wobei zumindest ein Datenträger 57 am Linsenträger 50 angeordnet ist. Am Datenträger 57 bzw. am Speichermedium sind Daten zu der Intraokularlinse 51 gespeichert. Der Datenträger 57 ist ein codierter NFC-Chip, oder Barcode, QR-Code oder vergleichbar Speicherchips oder Strichcode, welcher beim Einlegen von der Steuerungseinrichtung 26 ausgelesen wird. Zusätzlich umfassen die Daten bzw. Parameter zumindest eine der folgenden Informationen, nämlich Parameter zur Echtheit bzw. Validität des Linsenträgers (Zertifikat), Name, Artikelnummer, Herstelldatum, Haltbarkeit, Seriennummer und Position im Strahlengang, Justagewerte, Kalibrierwerte und einen Fokuspunkt in Abhängigkeit von der Addition in der Intraokularlinse 51.

**Figur 16** zeigt ein Sehprüfsystem 120 zum postoperativen Überprüfen zumindest eines mit einer Intraokularlinse 51 versehenes Auges eines Probanden 18 mit einer Linsenträgeraufnahme 25 zum Aufnehmen eines ersten Linsenträgers 55, wobei die Linsenträgeraufnahme 55 in einer zentralen Sichtachse 21 des optischen Strahlengangs 22 von distal zu proximal aufeinanderfolgend zwischen einer Cornea-Optik 40 und einer Okular-Optik 45 gelagert ist. Es ist eine Kompensationslinse 56 zum Korrigieren der im optischen Strahlengang 21 angeordneten Cornea-Optik 40 und Okular-Optik 45 vorhanden, welche in der Linsenträgeraufnahme 25 angeordnet ist. Das Sehprüfsystem 120 weist keinen Linsenträger 50 mit einer Intraokularlinse 51 auf, ist aber ansonsten strukturell und funktionell gleich aufgebaut, wie das Sehprüfsystem 20 gemäss der **Figur 1** bis **Figur 3****.**

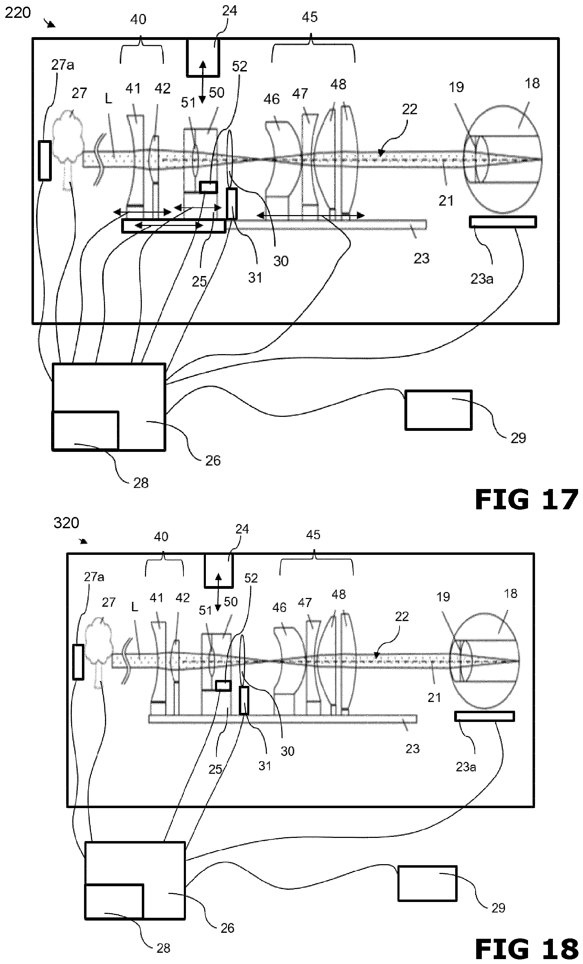

**Figur 17** zeigt eine weitere Ausführungsform eines Sehprüfsystems 220. Dieses Sehprüfsystem 220 umfasst zusätzlich zu dem in der **Figur 1** bis **Figur 3** offenbarten Sehprüfsystem 20 eine einstellbare Linse 30 zum Einstellen der Brechkraft, welche an der Trägereinrichtung 23 an einem Linsenträger 31 angeordnet ist. Diese ist im optischen Strahlengang 22 zusätzlich zur Verschiebung der Optiken 40, 45 und 51 angeordnet. Die einstellbare Linse 30 bzw. der Linsenträger 31 ist mit der Steuerungseinrichtung 26 zum Austausch von Daten bzw. Steuerbefehlen verbunden. Damit ist beim zuvor beschriebenen Verfahren 100 im Schritt b. ein Einstellen der Brechkraft mit der einstellbaren Linsen 30 zusätzlich möglich. Der Linsenträger 31 kann ein Speichermedium mit Daten aufweisen, wie zuvor für den Linsenträger 50 offenbart.

**Figur 18** zeigt eine weitere Ausführungsform eines Sehprüfsystems 320. Dieses Sehprüfsystem umfasst anstelle der Antriebseinrichtungen zum Verschieben der Corena-Optik gemäss des Sehprüfsystems 20 der **Figur 1** bis **Figur 3****,** eine einstellbare Linse 30 zum Einstellen der Brechkraft, welche an der Trägereinrichtung 23 an einem Linsenträger 31 angeordnet ist. Diese einstellbare Linse 30 ist mit der Steuerungseinrichtung 26 zum Austausch von Daten bzw. Steuerbefehlen verbunden. Damit ist beim zuvor beschriebenen Verfahren 100 im Schritt b. ein Einstellen der Brechkraft mit der einstellbaren Linsen 30 möglich, ohne dass ein Verschieben der Cornea-Optik bzw. des Linsenträgers 50 der Intraokularlinse 51 erfolgt.

Die dargestellte Position der einstellbaren Linse 30 in der **Figur 17** oder **Figur 18** auf der Trägereinrichtung 23 ist eine mögliche Variante. Die einstellbare Linse 31 kann auch zwischen der Anzeigeeinrichtung 27 und der Cornea-Optik 40 angeordnet sein, oder in der Okular-Optik 45 integriert sein, oder in der Cornea-Optik 40 integriert sein, oder zwischen der Okular-Optik 45 und dem Probanden 19 angeordnet sein.

### Bezugszeichenliste

- 18: Proband
- 19: Iris von 18
- 20: Sehprüfsystem
- 21: zentrale Sichtachse
- 22: optischer Strahlengang
- 23: Trägereinrichtung
- 23a: Kinn- oder Kopfstütze
- 24: Positioniereinrichtung
- 25: Linsenträgeraufnahme
- 26: Steuerungseinrichtung
- 27: Anzeigeeinrichtung
- 27a: Lichtquelle
- 28: Recheneinheit
- 29: Eingabegerät
- 30: einstellbare Linse
- 31: Linsenträger von 30

- 40: Cornea-Optik
- 41: Zerstreuungslinse von 40
- 42: Sammellinse von 40
- 43: Linsenoberfläche von 42
- 45: Okular-Optik
- 46: Korrekturlinse
- 46a: distale konkave Linsenoberfläche von 46
- 46b: distale konvexe Linsenoberfläche von 46
- 47: Zerstreuungslinse von 45
- 47a: Linsenoberfläche von 47
- 48: Sammellinse von 45
- 48a: Linsenoberfläche von 48

- 50: Linsenträger
- 51: erste Intraokularlinse
- 52: Speichermedium

- 55: Linsenträger
- 56: Kompensationslinse
- 57: Datenträger, Speichermedium

- 100: Verfahren
- 101-110: präoperative Verfahrensschritte
- 111: Implantation
- 112-119: postoperative Verfahrensschritte
- 120: Sehprüfsystem
- 220: Sehprüfsystem
- 320: Sehprüfsystem

- L: Lichtstrahl
- N: Nahbereich
- I: Intermediärbereich
- F: Fernbereich

## Patentansprüche

1. Verfahren zum Überprüfen zumindest eines Seheindrucks eines Probanden (18) mit einem Sehprüfsystem (20; 120; 220; 320), wobei das Sehprüfsystem (20; 120; 220; 320) eine Linsenträgeraufnahme (25) zum Aufnehmen zumindest eines Linsenträgers (50; 55) umfasst, die in einer zentralen Sichtachse (21) des optischen Strahlengangs (22) von distal zu proximal aufeinanderfolgend zwischen einer Cornea-Optik (40) und einer Okular-Optik (45) gelagert ist, umfassend zumindest die folgenden Schritte:
a. Positionieren eines ersten Linsenträgers (50) mit einer ersten Intraokularlinse (51) in der Linsenträgeraufnahme (25) des Sehprüfsystems (20; 120; 220; 320);
b. Verschieben der Cornea-Optik (40) und der Linsenträgeraufnahme (25) mit der Intraokularlinse (51) entlang der zentralen Sichtachse (21), wobei die Okular-Optik (45) in einem fixen Abstand zu zumindest einem Auge des Probanden (18) verbleibt, und/oder Einstellen der Brechkraft mit einer einstellbaren Linse (31);
c. Erfassen zumindest einer für den Seheindruck indikativen Information;
d. Zuordnen der mindestens einen indikativen Information zur ersten Intraokularlinse (51).

2. Verfahren nach Anspruch 1, **dadurch gekennzeichnet, dass** mindestens ein Datenwert, der während eines ersten Sehtests mit einem Objekt repräsentativ ist, gemessen wird, wobei insbesondere der mindestens eine Datenwert zur ersten Intraokularlinse (51) zugeordnet wird.

3. Verfahren nach Anspruch 1 oder 2, wobei das Sehprüfsystem (20; 120; 220; 320) eine Steuerungseinrichtung (26) umfasst und der erste Linsenträger (51; 55) ein Speichermedium (52; 57) umfasst, **dadurch gekennzeichnet, dass** ein erster Parameter von dem Speichermedium (52;57) des ersten Linsenträgers (51; 55) an die Steuerungseinrichtung (26) übermittelt wird, wobei die Steuerungseinrichtung (26) insbesondere zumindest das Verschieben der Linsenträgeraufnahme (25) entlang der zentralen Sichtachse (21) bewirkt.

4. Verfahren nach Anspruch 2 oder 3, wobei das Sehprüfsystem (20; 120; 220; 320) eine Recheneinheit (28) umfasst, **dadurch gekennzeichnet, dass** der mindestens eine Datenwert und/oder der erste Parameter in der Recheneinheit (28) verarbeitet wird.

5. Verfahren nach Anspruch 4, **dadurch gekennzeichnet, dass** ein Auswahlalgorithmus in der Recheneinheit (28) zumindest einen Datenwert zur ersten Intraokularlinse (51) mit einem weiteren Datenwert einer weiteren Intraokularlinse (51) vergleicht.

6. Verfahren nach einem der vorgenannten Ansprüche, **dadurch gekennzeichnet, dass** mindestens ein weiterer Datenwert, der während eines zweiten Sehtests mit einem Objekt repräsentativ ist, gemessen wird.

7. Verfahren nach einem der vorgenannten Ansprüche, **dadurch gekennzeichnet, dass** nach dem Schritt d) eine Kompensationslinse (56) die Linsenträgeraufnahme (25) positioniert wird und zumindest ein Sehtest durchgeführt wird.

8. Verfahren nach einem der vorgenannten Ansprüche, wobei das Sehprüfsystem (20; 120; 220; 320) zumindest eine Lichtquelle (27a) umfasst, **dadurch gekennzeichnet, dass** die zumindest eine Lichtquelle (27a) in den optischen Strahlengang (21) des Sehprüfsystem (20; 120; 220; 320) während eines Sehtests eingeblendet wird.

9. Verfahren nach einem der vorgenannten Ansprüche, wobei ein Eingabegerät vorhanden ist, **dadurch gekennzeichnet, dass** der Probanden (18) das Eingabegerät (29) während zumindest eines Sehtests bedient, um zumindest einen der gemessenen Datenwerte zu zuordnen.

10. Verfahren nach einem der vorgenannten Ansprüche, **dadurch gekennzeichnet, dass** zumindest eine Defokuskurve aufgenommen wird.

11. Verfahren nach einem der vorgenannten Ansprüche, wobei eine Positioniereinrichtung (24) vorhanden ist, **dadurch gekennzeichnet, dass** zumindest der erste Linsenträger (50) mit der Intraokularlinse (51) in die Linsenträgeraufnahme (25) positioniert wird, wobei insbesondere die Steuerungseinrichtung (26) Daten an die Positioniereinrichtung (24) übermittelt.

12. Sehprüfsystem (20; 120; 220; 320) zum Überprüfen zumindest eines Seheindrucks eines Probanden (18) mit einer Linsenträgeraufnahme (25) zum Aufnehmen eines ersten Linsenträgers (50, 55), wobei die Linsenträgeraufnahme 0 (25) in einer zentralen Sichtachse (21) von distal zu proximal aufeinanderfolgend zwischen einer Cornea-Optik (40) und einer Okular-Optik (45) gelagert ist, und eine Steuerungseinrichtung (26) vorhanden ist, welche ausgebildet ist zumindest ein Verfahren zum Überprüfen zumindest eines Seheindrucks eines Probanden (18) nach einem der Ansprüche 1 bis 11 durchzuführen.

13. Linsenträger (50) mit zumindest einer Intraokularlinse (51), wobei zumindest ein Datenträger (57) am Linsenträger (50) angeordnet ist, **dadurch gekennzeichnet, dass** am Datenträger (57) Daten, insbesondere Kalibierdaten und/oder Justagedaten, zu der Intraokularlinse (51) und/oder Linsenträger (50) gespeichert sind.

14. Computerprogrammprodukt, umfassend Befehle, die bei der Ausführung des Programms durch einen Computer eines Sehprüfsystems (20; 120; 220; 320), den Computer des Sehprüfsystems (20; 120; 220; 320) veranlassen, das Verfahren nach Anspruch 1 bis 11 auszuführen.

15. Computerlesbarer Datenträger, auf dem das Computerprogrammprodukt nach Anspruch 14 gespeichert ist.
